# EUROPÄISCHE PATENTSCHRIFT

(11) **EP 3 854 436 B1**
(45) Veröffentlichungstag und Bekanntmachung des Hinweises auf die Patenterteilung: **16.02.2022**
(21) Anmeldenummer: 21150884.1
(22) Anmeldetag: 11.01.2021
(51) Int. Cl.: A61M 16/20, A61M 39/22

(54) **VENTIL-ANORDNUNG MIT ABSPERREINHEIT FÜR EIN BEATMUNGSGERÄT**
VALVE ASSEMBLY WITH SHUT-OFF UNIT FOR A BREATHING DEVICE
AGENCEMENT DE SOUPAPE POURVU D'UNITÉ D'ARRÊT POUR UN APPAREIL RESPIRATOIRE

(30) Priorität: 21.01.2020 DE 102020000335
(43) Veröffentlichungstag der Anmeldung: 28.07.2021
(73) Patentinhaber: Drägerwerk AG & Co. KGaA, 23558 Lübeck (DE)
(72) Erfinder: Wolf, Andreas, 23558 Lübeck (DE)

(56) Entgegenhaltungen:
- WO-A1-2015/014899
- DE-A1- 1 960 093
- US-A1- 2014 058 524

## Beschreibung

Die Erfindung betrifft eine Ventil-Anordnung, welche in einer Fluidverbindung, z.B. in einem Beatmungskreislauf, zwischen einem Beatmungsgerät und einem Patienten angeordnet ist oder sich dort anordnen lässt, wobei das Beatmungsgerät den Patienten künstlich beatmet und wobei die Ventil-Anordnung sich bei einer bestimmten Überdruck-Situation automatisch öffnet. Die Erfindung betrifft weiterhin eine Beatmungs-Anordnung, welche ein Beatmungsgerät und eine erfindungsgemäße Ventil-Anordnung umfasst.

Mehrere Ventil-Anordnungen für medizinische Anwendungen mit Überdruckventilen sind bekannt geworden.

In US 2015/0 306 329 A1 wird eine Anordnung beschrieben, die den Druck oder den Volumenfluss in einem Patientenkreislauf begrenzt. Die Anordnung lässt sich mit einem endotrachealen Katheter (endotracheal tube 113) verbinden und umfasst ein proximal end 110 mit einem Adapter 109. In einem Adapter 409, 501 ist ein Überdruckventil (relief valve 205) angeordnet, in einem Adapter 306 ein Drosselventil (throttling valve 309). Mithilfe eines Betätigungselements (valve control surface 304, groove 305, stem 303) lässt sich das Drosselventil 309 verstellen und vermag dadurch die Querschnittsfläche einer Durchflussöffnung (aperture 301) zu verstellen. Falls der Druck in dem Patientenkreislauf (genauer: in dem hollow main body 213) eine Grenze übersteigt, so öffnet sich das Überdruckventil 205 gegen die Kraft eines Rückhaltemechanismus 208.

DE 699 11 704 T2 zeigt ein Beatmungsgerät, welches sich wahlweise in der Betriebsart "künstliche Beatmung" oder in der Betriebsart "Atmungsunterstützung" betreiben lässt. In der Betriebsart "künstliche Beatmung" wird Atemgas von einer Quelle 25 über eine Verbindung 23 in ein Rohr 4 geleitet, welches Atemgas zu einem Patienten leitet. Eine Leitung 12, die in das Rohr 4 führt, ist gesperrt. In der Betriebsart "Atmungsunterstützung" werden die Leitung 12 geöffnet und die Verbindung 23 gesperrt. Mithilfe des Ventils 29 wird die Zufuhr von Atemgas in die Leitung 12 gesteuert. Ein Begrenzer 30 begrenzt den Volumenstrom und / oder den Druck des zugeführten Atemgases.

In DE 102 40 992 B4 wird ein Überdruckventil 1 für Beatmungsgeräte beschrieben. Ein beweglicher Ventilteller 6 liegt auf einem Ventilsitz einer Atemgasleitung auf. Wenn das Überdruckventil 1 in der Betriebsart "manuelle Beatmung" betrieben wird, so vermag ein Überdruck das Überdruckventil 1 entgegen einer voreingestellten Schließkraft zu öffnen. Mithilfe eines Handrades 2 lässt sich die voreingestellte Schließkraft des Ventiltellers 6 verändern.

DE 20 2007 019 350 U1 und WO 2008 / 028228 A1 zeigen eine Ventilationsanordnung, die den Ausatmungsdruck absenkt, während ein Patient während einer CPAP-Therapie eine Atemmaske trägt und künstlich beatmet wird. Diese Ventilationsanordnung ist an einer Rohrleitung angeordnet, welche die Atemmaske mit einem Flussgenerator verbindet. In einer Ausgestaltung umfasst die Ventilationsanordnung ein Bedarfsventil, das sich öffnet, wenn der Patient einatmet oder Atemluft zu ihm gefördert wird, und sich schließt, wenn er ausatmet. Ein Überdruckventil an der Maske öffnet sich bei einem Überdruck an der Maske. In einer Ausgestaltung besitzt das Bedarfsventil eine Klappe, die sowohl in der geöffneten als auch in der geschlossenen Stellung arretiert wird. Die Arretierung in der geschlossenen Stellung der Klappe wird gelöst, und die Klappe öffnet sich, wenn der Ausatmungsdruck unter eine vorgegebene Schranke fällt.

In DE 1960093 A1 wird ein mit Überdruck und Strömungsabschaltung arbeitendes Beatmungsgerät beschrieben, welches einem Patienten Sauerstoff zuführt. Zwischen einer Gesichtsmaske 22 auf dem Gesicht des Patienten und einer Sauerstoffbombe 14 ist eine Fluidverbindung hergestellt, welche eine Gaszufuhr 12, ein Einatmungs-Gasrohr 20 und ein Ausatmungsventil-Steuerrohr 88 umfasst. Ein Ausatmungsventil 80 am Einatmungs-Gasrohr 20 umfasst einen Ringventilsitz 82 an einem Ausatmungs-Gasventilteil 78 sowie eine elastische Ventilmembran 86, die teleskopartig über ein Ausatmungsventil-Steuerrohr 88 geschoben ist. Die aktuelle Einstellung des Ventilmembran 86 wird durch die Strömung des Einatmungsgases entweder in das Ausatmungsventil-Steuerrohr oder aus dem Steuerrohr 88 durch einen Ausatmungsventil-Steuerstutzen 44 gesteuert.

In der nachveröffentlichten Offenlegungsschrift DE 10 2018 008 495 A1 wird eine Beatmungsschlauchvorrichtung 10 beschrieben, die einen Patienten mit einer Beatmungsvorrichtung 110 verbindet. In der Beatmungsschlauchvorrichtung 10 ist ein Druckbegrenzungselement 30 angeordnet. Dieses Druckbegrenzungselement 30 lässt sich zwischen einer Fließposition 31 und einer Blockierposition 32 hin- und her bewegen.

Der Erfindung liegt die Aufgabe zugrunde, eine Ventil-Anordnung für ein Beatmungsgerät bereitzustellen, wobei die Ventil-Anordnung und das Verfahren in unterschiedlichen Situationen eine höhere Betriebssicherheit als bekannte Ventil-Anordnungen und Verfahren bieten.

Die Aufgabe wird durch eine Ventil-Anordnung mit den Merkmalen des Anspruchs 1 gelöst. Vorteilhafte Ausgestaltungen sind in den Unteransprüchen angegeben.

Die erfindungsgemäße Ventil-Anordnung ist dazu ausgestaltet, in einem Fluidführungssystem eingesetzt zu werden, insbesondere in einem System mit mindestens einem Schlauch und / oder mindestens einer Röhre und / oder mindestens einem Tubus. Dieses Fluidführungssystem vermag eine Fluidverbindung zwischen einer patientenseitigen Koppeleinheit und einem Beatmungsgerät herzustellen, wobei durch die Fluidverbindung Fluid in beide Richtungen fließen kann. Ein Anästhesiegerät, welches ein Anästhesiemittel bereitstellt, ist ein Sonderfall eines Beatmungsgeräts im Sinne der Ansprüche.

Die patientenseitige Koppeleinheit lässt sich mit einem Patienten verbinden, sodass eine Fluidverbindung zwischen dem Beatmungsgerät und dem Patienten hergestellt werden kann, wenn der Patient mit der patientenseitigen Koppeleinheit verbunden ist. Insbesondere lässt die patientenseitige Koppeleinheit sich auf das Gesicht des Patienten legen oder in seinen Körper einführen.

Die erfindungsgemäße Ventil-Anordnung umfasst einen patientenseitigen Anschluss und einen geräteseitigen Anschluss. Mithilfe einer geeigneten geräteseitigen Fluidführungseinheit lässt sich eine Fluidverbindung zwischen dem geräteseitigen Anschluss der Ventil-Anordnung und einem Beatmungsgerät herstellen. Mithilfe einer geeigneten patientenseitigen Fluidführungseinheit lässt sich eine Fluidverbindung zwischen dem patientenseitigen Anschluss der Ventil-Anordnung und einer patientenseitigen Koppeleinheit für einen Patienten, den das Beatmungsgerät künstlich beatmen soll, herstellen. Die erfindungsgemäße Ventil-Anordnung kann auch in einer einzigen Fluidführungseinheit zwischen der patientenseitigen Koppeleinheit und dem Beatmungsgerät angeordnet sein, wobei die beiden Anschlüsse der Ventil-Anordnung in diese Fluidführungseinheit integriert sind.

Eine Fluidführungseinheit im Sinne der Ansprüche vermag ein Fluid zwischen zwei Punkten zu führen und einen Austritt des Fluids zwischen den beiden Punkten weitgehend zu verhindern, ohne notwendigerweise selber das Fluid zu bewegen. Ein Fluidführungssystem im Sinne der Ansprüche umfasst mindestens eine Fluidführungseinheit.

Eine Absperreinheit der erfindungsgemäßen Ventil-Anordnung lässt sich zwischen mindestens einer Öffnungsstellung und einer Verschlussstellung hin und her bewegen, optional zwischen jeder von mehreren möglichen Öffnungsstellungen und der Verschlussstellung. Wenn die Absperreinheit in der oder einer Öffnungsstellung ist, so ist eine Fluidkommunikation zwischen den beiden Anschlüssen der Ventil-Anordnung hergestellt oder lässt sich herstellen. Bei hergestellter Fluidkommunikation vermag ein Fluid von dem einen Anschluss zu dem anderen Anschluss zu fließen. Diese Fluidkommunikation ist unterbrochen oder - verglichen mit der oder jeder Öffnungsstellung - wenigstens eingeschränkt, d.h. die Flussrate von Fluid ist reduziert, wenn die Absperreinheit in der Verschlussstellung ist.

Die Arretiereinheit lässt sich in einen Öffnungs-Arretierzustand und in einen Verschluss-Arretierzustand überführen, also in einen von zwei verschiedenen Arretierzuständen. Im Öffnungs-Arretierzustand arretiert die Arretiereinheit die Absperreinheit in der oder einer Öffnungsstellung. Im Verschluss-Arretierzustand arretiert die Arretiereinheit die Absperreinheit in der Verschlussstellung. Um also die Absperreinheit aus einer Stellung in die oder eine andere Stellung zu bewegen, muss eine der Bewegung entgegenwirkende arretierende Wirkung der Arretiereinheit überwunden werden, beispielsweise eine entgegenwirkende Arretierkraft. Oder ein Arretierkörper der Arretiereinheit muss aus einer Arretierposition in eine Freigabeposition bewegt werden, wobei in der Freigabeposition der Arretierkörper die Verschlusseinheit nicht mehr arretiert.

Wenn die Absperreinheit in der Verschlussstellung ist und wenn außerdem am patientenseitigen Anschluss ein Druck oberhalb einer vorgegebenen Druckschranke anliegt, so bewegt die Ventil-Anordnung automatisch die Absperreinheit in die oder eine Öffnungsstellung, und zwar gegen eine arretierende Wirkung der Arretiereinheit.

Im Folgenden wird dargelegt, welche möglichen Vorteile der Erfindung in vielen Situationen auftreten können.

Erfindungsgemäß lässt sich die Absperreinheit zwischen der oder mindestens einer Öffnungsstellung und einer Verschlussstellung hin und her bewegen. In der oder jeder Öffnungsstellung ermöglicht die Absperreinheit, dass eine Fluidverbindung zwischen dem Beatmungsgerät und der patientenseitigen Koppeleinheit hergestellt oder herstellbar ist und ein Fluid durch die Ventil-Anordnung fließt. In der Verschlussstellung unterbricht die Absperreinheit diese Fluidverbindung oder reduziert wenigstens die Fließrate durch das Fluidführungssystem verglichen mit jeder Öffnungsstellung. In der oder mindestens einen Öffnungsstellung ermöglicht die Absperreinheit also eine künstliche Beatmung eines Patienten, bei der ein Fluid vom Beatmungsgerät zum Patienten fließt. In der Verschlussstellung trennt die Absperreinheit bevorzugt die patientenseitige Koppeleinheit vom Beatmungsgerät und von der Umgebung ab.

In vielen Fällen ist es von Zeit zu Zeit erforderlich, die Fluidverbindung zwischen der patientenseitigen Koppeleinheit und dem Beatmungsgerät zeitweise zu trennen, beispielsweise weil der Patient transportiert werden soll, das Beatmungsgerät oder das Fluidführungssystem gereinigt oder gewartet werden müssen oder weil Betriebsmittel ergänzt oder erneuert werden müssen. Die patientenseitige Koppeleinheit, z.B. ein Beatmungstubus oder ein Katheter oder eine Gesichtsmaske, ist mit dem Patienten verbunden und soll in der Regel auch nach der Trennung vom Beatmungsgerät mit dem Patienten verbunden bleiben, um den Patienten so wenig wie möglich zu belasten. Die Fluidverbindung muss auch dann getrennt werden, wenn der Patient von einem ersten Beatmungsgerät zu einem zweiten Beatmungsgerät transportiert wird, beispielsweise weil das erste Beatmungsgerät ein Transport-Beatmungsgerät ist, welches den Patienten an Bord eines Rettungsfahrzeugs und / oder auf einer Trage beatmet, der künstlich beatmete Patient zu einem Krankenhaus transportiert wird und anschließend das zweite Beatmungsgerät den Patienten in dem Krankenhaus beatmet. Auch in diesem Fall soll der Patient mit der patientenseitigen Koppeleinheit verbunden bleiben.

Wenn die Absperreinheit in der Verschlussstellung ist, lässt die Fluidverbindung zwischen dem Patienten und dem Beatmungsgerät sich trennen, ohne dass dadurch eine Fluidverbindung zwischen dem Patienten und der Umgebung hergestellt wird. Eine solche Fluidverbindung des Patienten mit der Umgebung ist in vielen Fällen unerwünscht, insbesondere deshalb, weil dann ein verbleibender Luftdruck (positive end-expiratory pressure, PEEP) in der Lunge des Patienten zu gering werden und die Lunge in sich zusammenfallen kann. Oft dauert es Stunden, um anschließend die Lunge wieder in einen funktionsfähigen Zustand zu verbringen. Das "Aufblasen" der Lunge übt zwangsläufig Scherkräfte auf die Alveolen (Lungenbläschen) aus, was diese abnutzt und dadurch schädigt. Die Arretiereinheit in dem Verschluss-Arretierzustand reduziert das Risiko, dass die Absperreinheit sich öffnet und dadurch die Fluidverbindung zur Umgebung ungewollt hergestellt wird.

Dank der Absperreinheit ist es nicht erforderlich, die Fluidverbindung manuell zu trennen und dann zu verschließen, beispielsweise indem jemand einen Schlauch abzieht und einen Stopfen setzt. Die Absperreinheit lässt sich so ausgestalten, dass die Absperreinheit in der Verschlussstellung die Fluidverbindung im Wesentlichen vollständig unterbricht, zumindest für die Zeitspanne, in welcher der Patient nicht künstlich beatmet wird. Diese Zeitspanne beträgt in der Regel nur wenige Minuten. Der Schritt, die Absperreinheit in die Verschlussstellung zu bewegen, erfordert oft weniger Zeit als andere mögliche Vorgehensweisen, um die Fluidverbindung zwischen dem Beatmungsgerät und dem Patienten zu unterbrechen, und reduziert das Risiko, dass eine unerwünschte Fluidverbindung der Lunge zur Umgebung auftritt.

Erfindungsgemäß arretiert die Arretiereinheit die Absperreinheit, und zwar sowohl in der oder jeder möglichen Öffnungsstellung oder wenigstens in einer von mehreren möglichen Öffnungsstellungen als auch in der Verschlussstellung. Die Arretiereinheit stellt also eine bistabile Arretierung für die Absperreinheit bereit. Diese bistabile Arretierung verringert das Risiko, dass die Absperreinheit sich ungewollt öffnet oder ungewollt schließt, was ohne eine erfindungsgemäße Arretierung beispielsweise aufgrund von Vibrationen oder Erschütterungen oder auch aufgrund von Berührungen oder Fehlhandlungen eines Benutzers geschehen kann. Bei einem ungewollten Öffnen kann so wie gerade beschrieben eine unerwünschte Fluidverbindung zwischen der Lunge des Patienten und der Umgebung auftreten. Bei einem ungewollten Schließen könnte es passieren, dass der Patient nicht ausreichend mit Luft versorgt wird.

Erfindungsgemäß öffnet die Absperreinheit sich bei einem ausreichend großen Druck am patientenseitigen Anschluss. In der Regel bringt der Patient diesen Druck auf. Weil die Absperreinheit erst bei einem Druck oberhalb der Druckschranke geöffnet wird, verhindert die Arretiereinheit, dass sich die Absperreinheit ungewollt dann öffnet, wenn der Patient nur leicht ausatmet oder hustet und der Druck am patientenseitigen Anschluss daher unter der Druckschranke bleibt.

Außerdem lässt sich die Ventil-Anordnung so ausgestalten, dass die arretierte Absperreinheit in der Verschlussstellung verbleibt, egal wie groß der Druck am geräteseitigen Anschluss ist, und sich nicht von allein öffnet, sondern nur nach einer Dearretierung. Diese Ausgestaltung verringert die Gefahr, dass die Absperreinheit sich ungewollt öffnet. Eine Dearretierung wird nämlich in der Regel gewollt durchgeführt.

Insbesondere aus folgendem Grund ist eine ungewollt geöffnete Absperreinheit ungewollt: Bei unterbrochener Fluidverbindung und geöffneter Absperreinheit könnte die Lunge des Patienten in einer Fluidverbindung mit der Umgebung stehen, was wie oben dargelegt unerwünscht ist. Bei hergestellter Fluidverbindung und ungewollt geöffneter Absperreinheit könnte der Patient einem hohen Druck vom geräteseitigen Anschluss her oder einem nicht für die künstliche Beatmung vorgesehenen Fluid ausgesetzt sein. Die Ventil-Anordnung lässt sich so ausgestalten, dass die Absperreinheit nur bei einem hohen Druck am patientenseitigen Anschluss oder überhaupt nicht automatisch öffnet und ansonsten arretiert ist und nur nach einem manuellen Eingriff dearretiert wird und dann bewegt werden kann. Diese Ausgestaltung erhöht die Betriebssicherheit sowohl bei hergestellter als auch bei fehlender Fluidverbindung mit einem Beatmungsgerät.

Außerdem ist in vielen Fällen eine relativ einfache mechanische Konstruktion der erfindungsgemäßen Ventil-Anordnung möglich. Die Ventil-Anordnung bewirkt, dass die Fluidverbindung in gewünschter Weise und betriebssicher geöffnet oder geschlossen ist, und stellt gleichzeitig einen automatisch wirkenden Überdruckmechanismus bereit, der nur bei einem hohen Druck am patientenseitigen Anschluss wirkt.

Erfindungsgemäß vermag die Ventil-Anordnung automatisch die Absperreinheit aus der Verschlussstellung in die oder eine Öffnungsstellung zu bewegen, und zwar gegen eine arretierende Wirkung der Arretiereinheit und dann, wenn am patientenseitigen Anschluss ein Druck oberhalb der Druckschranke anliegt. Dieses Merkmal reduziert die Gefahr, dass der Patient eine gesundheitliche Beeinträchtigung erleidet, insbesondere in folgender Situation: Der Patient ist mit einer patientenseitigen Koppeleinheit verbunden, und die Fluidverbindung zum Beatmungsgerät ist hergestellt oder zeitweise unterbrochen. Die Absperreinheit ist in der Verschlussstellung, und die Lunge des Patienten steht daher nicht in einer Fluidverbindung mit dem Beatmungsgerät und auch nicht in einer Fluidverbindung mit der Umgebung. Möglich ist, dass in dieser Situation der Patient hustet oder auf andere Weise kräftig ausatmet. Falls in dieser Situation die Absperreinheit in der Verschlussstellung bleiben würde, so würde patientenseitig ein hoher Druck und / oder ein hoher und / oder ein schneller Druckanstieg entstehen, und zwar oft schlagartig. Der resultierende hohe Druck / Druckanstieg kann sich bei geschlossener Absperreinheit nicht rasch genug abbauen und wirkt auf die Lunge ein. Diese Einwirkung kann zu einer Lungenschädigung, beispielsweise zu einem Barotrauma, führen. Erfindungsgemäß wird in dieser Situation automatisch die Absperreinheit geöffnet, und der Druck, der aufgrund des Hustens oder heftigen Ausatmens entsteht, kann durch die Ventil-Anordnung hindurch in die Umgebung entweichen. Die Gefahr ist daher deutlich geringer, dass die gerade beschriebene Lungenschädigung auftritt, als wenn die Absperreinheit geschlossen bliebe.

Die Ventil-Anordnung spricht automatisch an und bewegt die Absperreinheit automatisch in die Öffnungsstellung, wenn am patientenseitigen Anschluss ein Druck oberhalb der Druckschranke anliegt. Möglich, aber dank der Erfindung nicht erforderlich ist, dass der Patient selber oder jemand anders die Absperreinheit z.B. bei einem Husten des Patienten manuell öffnet. Die Gefahr einer Lungenschädigung wird auch dann reduziert, wenn der Patient die Absperreinheit nicht öffnen kann, beispielsweise weil er teilweise sediert ist, und keine sonstige Person das Husten bemerkt.

Erfindungsgemäß öffnet die Ventil-Anordnung die Absperreinheit, wenn am patientenseitigen Anschluss ein Druck oberhalb der Druckschranke anliegt. Diese Druckschranke lässt sich durch eine Konstruktion der Ventil-Anordnung festlegen. Die Druckschranke lässt sich so vorgegeben, beispielsweise durch eine entsprechende Ausgestaltung der Arretiereinheit, dass einerseits die Druckschranke unterhalb des anliegenden Drucks liegt, wenn bei geschlossener Absperreinheit der Patient ausreichend kräftig hustet oder anderweitig ausreichend kräftig ausatmet und dadurch einen Druck oberhalb der Druckschranke erzeugt. Bei geschlossener Absperreinheit würde die Gefahr einer Lungenschädigung bestehen. Die Ventil-Anordnung spricht automatisch an und öffnet automatisch die Absperreinheit, also ohne dass der Patient selbst oder eine andere Person den Patienten überwachen und bei Bedarf die Absperreinheit bewegen muss. Die Gefahr wird reduziert, dass die Lunge des Patienten durch einen starken Druck / Druckanstieg nach einem starken Husten geschädigt wird. Andererseits ist die Druckschranke hoch genug, um sicherzustellen, dass die Absperreinheit bei Erschütterungen, Vibrationen, Berührungen, ungewollten Fehlhandlungen oder einem nur leichten Husten oder Ausatmen des Patienten in der Verschlussstellung arretiert bleibt und daher ein ungewolltes Öffnen verhindert wird. Weil die Absperreinheit bei einem Druck unterhalb der Druckschranke in der Verschlussstellung bleibt, wird die Gefahr reduziert, dass der verbleibende Druck (PEEP) in der Lunge aufgrund von Vibrationen oder einem leichten Husten zu gering wird.

Das auslösende Ereignis, nämlich ein Druck oberhalb der Druckschranke am patientenseitigen Anschluss, lässt sich in vielen Fällen in einfacher Weise direkt dafür verwenden, um automatisch die Absperreinheit zu bewegen, insbesondere mittels einer einfachen mechanischen Ausgestaltung. Möglich, aber dank der Erfindung nicht erforderlich ist es, ein Husten des Patienten automatisch zu detektieren, z.B. durch einen entsprechenden pneumatischen oder elektrischen oder optischen oder akustischen Sensor. Der unerwünschte starke Druckanstieg beim Husten wird dank der erfindungsgemäßen Ventil-Anordnung verhindert, und zwar automatisch und ohne dass ein Mensch rasch reagieren muss.

Erfindungsgemäß ist der Druck am patientenseitigen Anschluss oberhalb der Druckschranke das auslösende Ereignis dafür, dass die Absperreinheit gegen die arretierende Wirkung in die Öffnungsstellung bewegt wird. Jedoch ist es zwar möglich, aber nicht erforderlich, dass der am patientenseitigen Anschluss anliegende Druck allein die arretierende Wirkung der Arretiereinheit überwindet. In vielen Fällen wird dieser Druck ausschließlich von einem spontan atmenden Patienten aufgebracht und ist daher begrenzt. Möglich ist, dass ein zusätzlicher Mechanismus der erfindungsgemäßen Ventil-Anordnung die arretierende Wirkung überwindet. Dadurch kann die Arretiereinheit eine größere arretierende Kraft ausüben (höhere Druckschranke), was die Betriebssicherheit erhöht, und dennoch kann ein spontan atmender Patient bewirken, dass die Absperreinheit bei Bedarf automatisch geöffnet wird, beispielsweise durch ein Husten.

Erfindungsgemäß ist die Absperreinheit in der Öffnungsstellung und in der Verschlussstellung arretiert. Außerdem überwindet die Ventil-Anordnung die arretierende Wirkung der Arretiereinheit, wenn die Absperreinheit in der Verschlussstellung ist und der am patientenseitigen Anschluss anliegende Druck oberhalb der Druckschranke ist. Insbesondere diese Merkmale unterscheidet die erfindungsgemäße Ventil-Anordnung von einer Anordnung mit einem üblichen Überdruckventil, welches sich typischerweise bei Überdruck gegen eine Rückstellkraft öffnet und bei geringerem Druck wieder schließt. Ein Überdruckventil kann auch ungewollt, z.B. bei Vibrationen oder Erschütterungen oder Berührungen, ansprechen und ist in keiner Stellung arretiert. Darüber hinaus lässt sich die erfindungsgemäße Ventil-Anordnung so ausgestalten, dass sie zwar bei einem ausreichend hohen Druck am patientenseitigen Anschluss automatisch die Absperreinheit öffnet, jedoch ansonsten die Absperreinheit nicht automatisch bewegt, egal wie groß der Druck am geräteseitigen Anschluss ist. Ein herkömmliches Überdruckventil an einer Fluidverbindung öffnet sich hingegen in der Regel bei ausreichend großem Druck, egal von welcher Seite dieser Überdruck anliegt.

Die Erfindung vermeidet die Notwendigkeit, ein herkömmliches Überdruckventil vorzusehen, um die Gefahr einer Lungenschädigung zu reduzieren. Weil die erfindungsgemäße Ventil-Anordnung einen Mechanismus bereitstellt, der vor einem unerwünschten Überdruck schützt, kann in vielen Fällen ein Überdruckventil eingespart werden, das räumlich von der Absperreinheit getrennt ist, was Bauraum und Gewicht einspart. Insbesondere ist es nicht erforderlich, an der patientenseitigen Koppeleinheit ein Überdruckventil anzubringen. Die Erfindung lässt sich auch in Kombination mit einem herkömmlichen Überdruckventil einsetzen.

Erfindungsgemäß öffnet die Ventil-Anordnung die Absperreinheit gegen die Arretierkraft, wenn am patientenseitigen Anschluss ein Druck oberhalb der Druckschranke anliegt. Insbesondere dieses Merkmal unterscheidet die erfindungsgemäße Ventil-Anordnung von einer beispielsweise aus DE 20 2007 019 350 U1 bekannten Anordnung mit einer Absperreinheit. Eine solche Anordnung lässt sich während einer künstlichen Beatmung einsetzen. Die Absperreinheit öffnet sich, wenn Luft zur patientenseitigen Koppeleinheit hin fließt, und schließt sich, wenn Luft von der patientenseitigen Koppeleinheit weg fließt. Die in DE 20 2007 019 350 U1 beschriebene Absperreinheit öffnet sich bei einem geringen Druck am patientenseitigen Anschluss, schließt sich bei einem höheren Druck wieder und lässt sich während einer künstlichen Beatmung verwenden, aber nicht dann, wenn die Fluidverbindung zwischen der patientenseitigen Koppeleinheit und dem Beatmungsgerät unterbrochen ist und eine unerwünschte Fluidverbindung zwischen der Lunge des Patienten und der Umgebung verhindert werden soll. Die erfindungsgemäße Ventil-Anordnung öffnet hingegen die Absperreinheit bei einem ausreichend hohen Druck am patientenseitigen Anschluss und lässt sich daher sowohl bei hergestellter als auch bei unterbrochener oder beendeter Fluidverbindung zwischen der patientenseitigen Koppeleinheit und dem Beatmungsgerät verwenden.

Bevorzugt umfasst die Ventil-Anordnung eine Fluidführungseinheit, welche die beiden Anschlüsse fluidleitend miteinander verbindet und welche bevorzugt in das Fluidführungssystem zwischen der patientenseitigen Koppeleinheit und dem Beatmungsgerät integriert ist. Die Absperreinheit in der Verschlussstellung verschließt diese Fluidführungseinheit vollständig oder wenigstens teilweise. Diese Ausgestaltung ermöglicht es, die beiden Anschlüsse der Ventil-Anordnung räumlich getrennt voneinander und getrennt von der Absperreinheit anzuordnen. Ermöglicht wird, die beiden Anschlüsse unabhängig voneinander an jeweils eine geltende Norm anzupassen.

Erfindungsgemäß wird die Absperreinheit automatisch aus der Verschlussstellung in die oder eine Öffnungsstellung überführt, wenn am patientenseitigen Anschluss ein Druck oberhalb der Druckschranke anliegt. In einer bevorzugten Ausgestaltung bewirkt dieses Ereignis automatisch, dass die Arretiereinheit die bewegte Absperreinheit nunmehr in dieser Öffnungsstellung arretiert. Dadurch wird verhindert, dass die Absperreinheit nach dem Öffnen unbeabsichtigt wieder geschlossen wird, was bei einem üblichen Rückstellelement oder einem üblichen Überdruckventil passieren kann. Die ungewollt wieder geschlossene Absperreinheit könnte zu einem zu hohen Druck auf die Lunge des Patienten führen. Wenn die Absperreinheit nicht in der oder einer Öffnungsstellung arretiert wäre, könnte sie wieder in die Verschlussstellung gelangen und dann den Versuch des Patienten behindern, nach dem Husten wieder einzuatmen. Weiterhin wird durch die Arretierung verhindert, dass ein Patient, der über eine längere Zeit hustet, ein Oszillieren der Absperreinheit zwischen zwei Positionen bewirkt, was oft ebenfalls unerwünscht ist, insbesondere wegen dem mechanischen Verschleiß und / oder einer möglichen Geräuschentwicklung.

Bevorzugt bleibt die Absperreinheit auch bei einem Druck oberhalb der Druckschranke, der am geräteseitigen Anschluss anliegt, in der Verschlussstellung. Dadurch wird die Gefahr reduziert, dass ein zu hoher Druck auf den Patienten einwirkt, insbesondere bei hergestellter Fluidverbindung.

Bevorzugt umfasst die Arretiereinheit eine mechanische oder pneumatische Feder und / oder einen Arretierkörper. Insbesondere mittels dieser Ausgestaltung lässt sich die Arretiereinheit so ausgestalten, dass sie weitgehend unabhängig von der Richtung der Schwerkraft relativ zur Ventil-Anordnung funktioniert. Diese Wirkung ist insbesondere deshalb von Vorteil, weil die Ventil-Anordnung in fast jeder Position relativ zum Patienten, relativ zum Beatmungsgerät und relativ zur Schwerkraft angeordnet sein kann und die Absperreinheit sich in der Öffnungsstellung insbesondere über, unter oder neben einer Fluidverbindung zwischen dem Patienten und dem Beatmungsgerät befinden kann. Falls die Orientierung der erfindungsgemäßen Ventil-Anordnung sich im Verlaufe eines Einsatzes ändert, beeinträchtigt dies nicht wesentlich die arretierende Wirkung.

In einer Ausgestaltung überführt der am patientenseitige Anschluss anliegende und in der Regel vom Patienten erzeugte Druck direkt die Absperreinheit gegen die arretierende Wirkung in die oder eine Öffnungsstellung, beispielsweise mithilfe eines Rückschlagventils, welches sich durch den anliegenden Druck bewegt und die Absperreinheit in die Öffnungsstellung überführt, wenn der anliegende Druck oberhalb der Druckschranke ist. Bei einem Druck gleich oder größer der Druckschranke wird also die arretierende Wirkung überwunden oder aufgehoben.

Möglich ist hingegen auch folgende Ausgestaltung der Arretiereinheit: Die Arretiereinheit arretiert in der Verschluss-Arretierposition auch dann die Absperreinheit, wenn der am patientenseitigen Anschluss anliegende Druck zwar größer oder gleich der Druckschranke ist, aber unterhalb einer größeren weiteren Druckschranke ist, oder auch bei jedem am patientenseitigen Anschluss anliegenden Druck. Dann ist in vielen Fällen die arretierende Wirkung groß und sicher genug, um ein ungewolltes Öffnen der Absperreinheit zu verhindern.

In einer bevorzugten Ausgestaltung der Erfindung umfasst die Ventil-Anordnung einen Überdruckmechanismus. Dieser Überdruckmechanismus spricht an, wenn die Absperreinheit in der Verschlussstellung ist und dann der am patientenseitigen Anschluss anliegende Druck oberhalb der Druckschranke liegt, und überführt in dieser Situation die Absperreinheit gegen die arretierende Wirkung in die Öffnungsstellung. Der Überdruckmechanismus vermeidet einen möglichen Nachteil, der entstehen könnte, wenn der vom Patienten erzeugte Druck direkt die Absperreinheit gegen die arretierende Wirkung bewegen müsste: Bei einer solchen direkten Kopplung ohne einen Überdruckmechanismus wäre in vielen Fällen entweder der Patient bei einem starken Husten gefährdet, weil die Druckschranke zu hoch ist, oder die Arretiereinheit übt keine ausreichende arretierende Wirkung aus, weil die Druckschranke zu niedrig ist, und die Absperreinheit könnte sich ungewollt öffnen.

Die Arretiereinheit im Verschluss-Arretierzustand hält die Absperreinheit in der Verschlussposition. Bevorzugt spricht der optionale Überdruckmechanismus automatisch an, wenn in dieser Situation der Druck am patientenseitigen Anschluss oberhalb der Druckschranke liegt. Der Überdruckmechanismus überwindet die arretierende Wirkung der Arretiereinheit. Dank des Überdruckmechanismus kann die Druckschranke einerseits so niedrig gewählt werden, dass ein Husten oder ein sonstiges heftiges Ausatmen des Patienten den Überdruckmechanismus auslöst und dieser automatisch die Absperreinheit öffnet. Andererseits kann die Druckschranke so hoch und insbesondere die Arretiereinheit so ausgestaltet werden, dass die arretierende Wirkung die Absperreinheit auch bei üblichen Vibrationen und Erschütterungen in der Verschlussposition hält. Der Überdruckmechanismus verstärkt praktisch die Kraft, die von dem am patientenseitigen Anschluss anliegenden Druck ausgeht. In einer Ausgestaltung aktiviert der am patientenseitigen Anschluss anliegende Druck den Überdruckmechanismus.

Bevorzugt nimmt der Überdruckmechanismus keinen Einfluss auf die Absperreinheit, solange der Druck am patientenseitigen Anschluss unterhalb der Druckschranke liegt oder wenn die Absperreinheit in der Öffnungsstellung ist. Insbesondere hängt der Überdruckmechanismus nicht davon ab, wie hoch der Druck am geräteseitigen Anschluss ist. Falls die Absperreinheit in der Verschlussstellung ist, so wäre es in vielen Fällen unerwünscht, dass ein ausreichend hoher Druck auf den geräteseitigen Anschluss die Absperreinheit öffnet und dieser Druck dann auf den Patienten einwirkt. Außerdem nimmt der Überdruckmechanismus bevorzugt keinen Einfluss auf die Absperreinheit, wenn diese in der Öffnungsstellung ist. Daher beeinträchtigt der Überdruckmechanismus in vielen Fällen nicht den Vorgang, dass das Beatmungsgerät den Patienten künstlich beatmet.

In einer Ausgestaltung umfasst der Überdruckmechanismus mindestens eine Feder, die bevorzugt durch eine Bewegung der Absperreinheit in die Verschlussstellung vorgespannt wird und in dem vorgespannten Zustand gehalten wird. Bei einem Druck am patientenseitigen Anschluss oberhalb der Druckschranke wird eine Einrastung oder sonstige Blockierung der vorgespannten Feder automatisch aufgehoben, und die sich entspannende Feder bewegt die Absperreinheit gegen die arretierende Wirkung in die oder eine Öffnungsstellung. Diese Ausgestaltung führt zu einem einfachen und bevorzugt rein mechanischen Überdruckmechanismus. Diese Feder kann eine Druckfeder oder eine Zugfeder sein und mechanisch oder auch pneumatisch arbeiten. Möglich sind mehrere parallel angeordnete Federn.

In einer Ausgestaltung lässt die Absperreinheit sich nur gegen die arretierende Wirkung der Arretiereinheit bewegen. In einer anderen Ausgestaltung lässt sich die Arretiereinheit aus mindestens einem Arretierzustand, bevorzugt aus jedem Arretierzustand, in einen optionalen Freigabezustand überführen und übt im Freigabezustand keine oder wenigstens nur eine geringere arretierende Wirkung aus - verglichen mit den beiden Arretierzuständen. Ohne einen Freigabezustand könnte folgende ungewünschte Situation auftreten: Eine relativ große Kraft ist erforderlich, um die arretierte Absperreinheit aus der einen Stellung in die andere Stellung zu bewegen. Aufgrund dieser Bewegung mit großer Kraft wird die Fluidverbindung ungewollt unterbrochen. Der Freigabezustand reduziert dieses Risiko.

Bevorzugt lässt sich die Absperreinheit mithilfe einer Betätigungseinheit aus der oder mindestens einer Öffnungsstellung in die Verschlussstellung und wieder zurück aus der Verschlussstellung in die Öffnungsstellung bringen, d.h. die Absperreinheit lässt sich manuell betätigen. Die Betätigungseinheit kann direkt an der Ventil-Anordnung angeordnet sein oder räumlich von der Ventil-Anordnung entfernt sein und beispielsweise über Funkwellen oder einem Kabel mit einem Stellglied für die Absperreinheit in einer Verbindung stehen. Dieses Merkmal ermöglicht es, rasch eine Fluidverbindung zu trennen und wieder herzustellen, wobei diese Fluidverbindung durch die Ventil-Anordnung hindurch führt. Nicht erforderlich ist es, eine patientenseitige Fluidführungseinheit von einem Beatmungsgerät zu trennen und nach dem Trennen mit einer speziellen Kappe oder gar mit der Hand zu verschließen, um eine unerwünschte Fluidverbindung zwischen dem Patienten und der Umgebung zu unterbinden. Ein Verschließen auf diese Weise ist häufig unhygienisch und / oder unterbindet die unerwünschte Fluidverbindung nicht sicher und / oder nicht ausreichend und / oder nicht rasch genug.

Möglich ist, dass die Ventil-Anordnung bei einem Druck oberhalb der Druckschranke automatisch die Absperreinheit in die Öffnungsstellung bewegt und zusätzlich die Arretiereinheit in den optionalen Freigabezustand überführt. Die Absperreinheit ist dann nicht mehr arretiert. Möglich ist auch, dass die Absperreinheit nach Ablauf einer vorgegebenen Zeitspanne automatisch wieder in die Verschlussstellung überführt wird.

In einer bevorzugten Ausgestaltung bewirkt die Ventil-Anordnung hingegen dann, wenn der Druck am patientenseitigen Anschluss oberhalb der Druckschranke ist, dass die Absperreinheit in die oder eine Öffnungsstellung bewegt wird und außerdem die Arretiereinheit in den Öffnungs-Arretierzustand überführt wird. Dadurch wird die Absperreinheit in der Öffnungsstellung arretiert. In einer Ausführungsform vermag der optionale Überdruckmechanismus diesen zusätzlichen Schritt durchzuführen. Die Ventil-Anordnung, also der am patientenseitigen Anschluss anliegende Druck und / oder optional der Überdruckmechanismus, bewirkt also, dass die Absperreinheit geöffnet wird, und zusätzlich, dass die Absperreinheit in der Öffnungsstellung arretiert wird. Dadurch wird verhindert, dass die Absperreinheit sich zufällig oder ungewollt wieder schließt, beispielsweise aufgrund von Vibrationen oder Erschütterungen oder Berührungen. Um die Absperreinheit wieder in die Verschlussstellung zu bringen, ist eine gewollte Bewegung der Absperreinheit, z.B. eine Betätigung der Betätigungseinheit, erforderlich.

In einer Ausgestaltung umfasst die Ventil-Anordnung ein manuell betätigbares Sicherheitselement, z.B. einen Knopf oder eine bewegliche Scheibe. Wenn das Sicherheitselement in einer Ruheposition ist, hält das Sicherheitselement die Arretiereinheit im Öffnungs-Arretierzustand. Bevorzugt hält das Sicherheitselement in derselben oder in einer anderen Ruheposition die Arretiereinheit im Verschluss-Arretierzustand. Nachdem ein Benutzer das Sicherheitselement betätigt und dadurch aus dem Ruhezustand heraus bewegt hat, wird die Arretiereinheit in den optionalen Freigabezustand überführt und arretiert nicht mehr die Absperreinheit, sondern gibt sie frei. Das Sicherheitselement reduziert also das Risiko, dass die Absperreinheit ungewollt dearretiert und als Folge ungewollt bewegt wird.

Diese Ausgestaltung lässt sich mit der oben beschriebenen Betätigungseinheit kombinieren In vielen Fällen vermeidet das Sicherheitselement die Notwendigkeit, die Betätigungseinheit gegen die arretierende Wirkung der Arretiereinheit bewegen zu müssen. Die Arretiereinheit kann in einem Arretierzustand eine größere Arretierkraft ausüben verglichen mit einer Ausgestaltung ohne Freigabezustand und ohne Sicherheitselement, was die Gefahr weiter reduziert, dass die Absperreinheit ungewollt bewegt wird. Andererseits ist eine geringere Kraft erforderlich, um die Absperreinheit manuell zu öffnen oder zu schließen, wenn das Sicherheitselement im Freigabezustand ist, verglichen mit einer Ausführungsform ohne Sicherheitselement. Bevorzugt hält ein Rückstellelement das Sicherheitselement in der Ruheposition und ist bestrebt, das Sicherheitselement aus der Freigabeposition wieder in die Ruheposition zu bewegen.

In einer Ausgestaltung umfasst die Ventil-Anordnung zusätzlich ein Überdruckventil. Falls am geräteseitigen Anschluss ein Druck oberhalb einer Überdruckschranke anliegt, so öffnet sich dieses Überdruckventil, bevorzugt gegen die Kraft eines Rückstellelements. Bevorzugt befindet dieses Überdruckventil sich zwischen der Absperreinheit, wenn diese in der Verschlussstellung ist, und dem geräteseitigen Ende der Ventil-Anordnung, z.B. im geräteseitigen Anschluss. Dieses Überdruckventil öffnet sich insbesondere dann, wenn bei geschlossener Absperreinheit das Beatmungsgerät Beatmungshübe ausführt. Das geöffnete Überdruckventil verhindert in vielen Fällen, dass die Absperreinheit ungewollt geöffnet und der Patient gefährdet wird. Weiterhin wird das Risiko reduziert, dass das Beatmungsgerät oder das Fluidführungssystem beschädigt wird. Bevorzugt schließt das Überdruckventil sich automatisch wieder, wenn der anliegende Druck geringer wird.

Erfindungsgemäß wird die Absperreinheit gegen die arretierende Wirkung der Arretiereinheit aus der Verschlussstellung in die oder eine Öffnungsstellung überführt, wenn am patientenseitigen Anschluss ein Druck oberhalb der Druckschranke anliegt. In einer Ausgestaltung ist diese Druckschranke fest durch die Konstruktion der Ventil-Anordnung vorgegeben.

In einer anderen Ausgestaltung umfasst die Ventil-Anordnung zusätzlich eine Justiereinheit zum Verändern der Druckschranke. Mithilfe dieser optionalen Justiereinheit kann ein Benutzer die Druckschranke manuell verändern und dadurch insbesondere die Ventil-Anordnung an einen Patienten anpassen. Diese Justiereinheit vermag beispielsweise die arretierende Wirkung der Arretiereinheit oder auch ein aktivierendes Element des oben beschriebenen optionalen Überdruckmechanismus zu verändern. Beispielsweise umfasst die Arretiereinheit eine mechanische oder pneumatische Feder, die eine arretierende Wirkung ausübt oder einen Arretierkörper der Arretiereinheit in einer Arretierposition hält. Mithilfe der Justiereinheit lassen die mechanische Spannung oder eine pneumatische Eigenschaft dieser Feder sich verändern.

Möglich ist, dass die optionale Justiereinheit eine kontinuierliche Veränderung der Druckschranke innerhalb eines vorgegebenen Bereichs ermöglicht. In einer bevorzugten Ausgestaltung rastet die Justiereinheit ein, wenn die Druckschranke auf einen von mehreren möglichen Werten eingestellt ist, und lässt sich nur mit einer gewissen Krafteinwirkung weiter bewegen. Diese bevorzugte Ausgestaltung verhindert, dass die Druckschranke ungewollt auf einen zu hohen oder auf einen zu niedrigen Wert eingestellt wird, was ohne ein Einrasten passieren könnte, wenn die Justiereinheit ungewollt bewegt oder auf andere Weise ungewollt betätigt wird.

In einer Ausgestaltung vermag die Arretiereinheit die Absperreinheit in genau einer Verschlussstellung und in genau einer Öffnungsstellung zu arretieren. Bevorzugt sind diese beiden Stellungen die beiden möglichen Endpositionen der Absperreinheit und besonders bevorzugt auch die beiden Endpositionen der optionalen Betätigungseinheit. In einer anderen Ausgestaltung vermag die Arretiereinheit die Absperreinheit zusätzlich in mindestens einer Zwischenstellung zwischen der Verschlussstellung und der Öffnungsstellung zu arretieren. Auch in dieser Zwischenstellung ist eine Fluidkommunikation zwischen den beiden Anschlüssen möglich, aber mit einer geringeren Fließrate als in der vollständig geöffneten Öffnungsstellung. Diese Ausgestaltung ermöglicht es, dass die Absperreinheit dann, wenn der Druck am patientenseitigen Anschluss oberhalb der Druckschranke liegt, wahlweise in die oder eine Zwischenstellung oder in die Öffnungsstellung überführt wird, je nachdem wie groß dieser Druck ist. Auch in der Zwischenstellung ist die Absperreinheit arretiert und kann nicht ungewollt bewegt werden. Auch in der Zwischenstellung kann Druck vom patientenseitigen Anschluss her abgebaut werden.

Erfindungsgemäß wird die Absperreinheit automatisch aus der Verschlussstellung in die oder eine Öffnungsstellung bewegt, wenn am patientenseitigen Anschluss ein Druck oberhalb der vorgegebenen Druckschranke anliegt. In einer Ausgestaltung ist die optionale Betätigungseinheit dergestalt mit der Absperreinheit verbunden, dass folgende Effekt bewirkt wird: Die Betätigungseinheit zeigt durch ihre aktuelle Stellung an, in welcher Position sich die Absperreinheit aktuell befindet. Eine manuell bewirkte Betätigung der Betätigungseinheit bewegt die Absperreinheit. Umgekehrt bewegt eine automatisch aufgrund des Drucks, der am patientenseitigen Anschluss anliegt, hervorgerufene Bewegung der Absperreinheit, dass die Betätigungseinheit bewegt wird. Beispielsweise steht eine Längsachse der Betätigungseinheit senkrecht zur Fluidrichtung von Fluid durch die Ventil-Anordnung, wenn die Absperreinheit in der oder der maximal möglichen Verschlussstellung ist, und ist parallel zur Flussrichtung bei der Absperreinheit in der Öffnungsstellung. Diese Ausgestaltung zeigt auf besonders intuitive Weise an, ob die Absperreinheit in der Verschlussstellung oder in der Öffnungsstellung ist.

In einer Ausgestaltung löst das Ereignis, dass die Absperreinheit aufgrund eines am patientenseitigen Anschluss anliegenden Drucks in die Öffnungsstellung bewegt wird, den Schritt aus, dass automatisch ein Alarm erzeugt und in einer von einem Menschen wahrnehmbaren Weise ausgegeben wird. Insbesondere wird durch den Alarm ein Mensch informiert, falls der Patient hustet oder kräftig ausatmet und dadurch ein Öffnen der Absperreinheit bewirkt hat. Ein am patientenseitigen Anschluss anliegender Druck oberhalb der Druckschranke lässt sich häufig leichter detektieren als direkt ein Husten des Patienten. In einer Ausgestaltung wird dieser Alarm kabelgebunden oder drahtlos, z.B. per Funkwellen, an einen räumlich entfernten Empfänger übertragen und von diesem Empfänger oder auf einer separaten Ausgabeeinheit ausgegeben.

Beispielsweise detektiert ein Ereignissensor automatisch das Ereignis, dass der am patientenseitige Anschluss anliegende Druck oberhalb der Druckschranke liegt, was den Alarm auslöst. Oder der Ereignissensor detektiert automatisch das Ereignis, dass die Absperreinheit geöffnet ist. Der Ereignissensor löst den Alarm aus.

In einer Ausgestaltung ist in einer Fluidverbindung zwischen der Ventil-Anordnung und dem Beatmungsgerät ein Filter angeordnet, insbesondere ein Filter für Viren, Mikroben und / oder Flüssigkeitstropfen. Dieser Filter verhindert, dass Viren und Mikroben durch die Fluidverbindung in das Beatmungsgerät gelangen. Die Ventil-Anordnung ermöglicht es, die Absperreinheit zu schließen und in der Verschlussstellung zu arretieren, den Filter auszutauschen und dann die Absperreinheit wieder zu öffnen und in der Öffnungsstellung zu arretieren. Auch in dieser Situation wird die Absperreinheit automatisch geöffnet, wenn am patientenseitigen Anschluss ein Druck oberhalb der Druckschranke anliegt, beispielsweise weil der Patient hustet.

Die Ausgestaltung mit dem Filter lässt sich mit der gerade beschriebenen Ausgestaltung, dass ein Alarm erzeugt und ausgegeben wird, kombinieren. Dieser Alarm kann einen Anlass für einen Menschen sein, den Filter zu überprüfen. Möglich ist nämlich, dass der Filter aufgrund des Hustens verstopft wird und ausgetauscht werden muss.

In einer Ausgestaltung umfasst die Ventil-Anordnung zusätzlich einen Drucksensor. Dieser Drucksensor misst den Druck, der aktuell am patientenseitigen Anschluss anliegt. Der Drucksensor misst den anliegenden Druck mindestens dann, wenn die Absperreinheit in der Verschlussstellung ist. Beispielsweise misst der Drucksensor ein Maß für eine Kraft, die aus Richtung des patientenseitigen Anschlusses her auf die Absperreinheit in der Verschlussstellung einwirkt. Die Absperreinheit und der Drucksensor zusammen ermöglichen es, die Absperreinheit kurzzeitig zu verschließen und bei verschlossener Absperreinheit den von Patienten ausgehenden und am patientenseitigen Anschluss anliegenden Druck zu messen. Dieser gemessene Druck ist ein Maß für die spontane Atmung (eigene Atmungsaktivität) des Patienten. Dieses Maß für die spontane Atmung lässt sich dafür verwenden, das Beatmungsgerät zu regeln. Auch bei dieser Anwendung ist sichergestellt, dass die Absperreinheit bei Bedarf, z.B. wenn der Patient hustet, automatisch geöffnet wird.

Bevorzugt wird der am patientenseitigen Anschluss anliegende und gemessene Druck in einer von einem Menschen wahrnehmbaren Form ausgegeben und / oder an einen räumlich entfernten Empfänger übermittelt, beispielsweise kabelgebunden oder drahtlos. Bevorzugt zeigt eine Anzeigeeinheit am Beatmungsgerät oder an der Ventil-Anordnung den gemessenen Druck an. Diese Ausgestaltung erleichtert es, den aktuellen Zustand des künstlich beatmeten Patienten auch aus der Ferne zu überwachen.

In einer Ausgestaltung umfasst die Ventil-Anordnung zusätzlich zum Drucksensor eine Sendeeinheit. Diese Sendeeinheit vermag einen Messwert vom Drucksensor zu empfangen und den empfangenen Messwert an einen räumlich entfernten Empfänger zu übermitteln. Insbesondere vermag die Sendeeinheit den empfangenen Messwert per Funkwellen zu übermitteln.

Erfindungsgemäß lässt die Absperreinheit sich aus der oder einer Öffnungsstellung in eine Verschlussstellung bewegen, in welcher die Absperreinheit die Fluidverbindung unterbricht oder wenigstens einschränkt. Der geräteseitige Anschluss der Ventil-Anordnung ist mit einer Fluidführungseinheit verbunden oder lässt sich verbinden. Beispielsweise verbindet diese Fluidführungseinheit die Ventil-Anordnung mit einem Beatmungsgerät. In einer Ausgestaltung ist diese Verbindung mechanisch mit der Absperreinheit verbunden, und zwar so, dass folgende Wirkung erzielt wird: Der Schritt, dass die Absperreinheit in die Verschlussstellung bewegt wird, löst automatisch die Verbindung zwischen dem geräteseitigen Anschluss und der Fluidführungseinheit. Beispielsweise werden eine Schnapphalterung oder eine Federhalterung geöffnet.

Die Erfindung betrifft weiterhin eine Fluidführungs-Anordnung, welche eine erfindungsgemäße Ventil-Anordnung und eine Fluidführungseinheit umfasst. Der geräteseitige Anschluss der Ventil-Anordnung ist lösbar mit der Fluidführungseinheit verbunden und steht wenigstens zeitweise mit der Fluidführungseinheit in einer Fluidverbindung. Bevorzugt wird die Fluidführungseinheit von der Ventil-Anordnung gelöst, wenn die Absperreinheit in die Verschlusseinrichtung bewegt wird, besonders bevorzugt so wie im vorigen Absatz beschrieben.

In einer bevorzugten Anwendung verbindet die Fluidführungseinheit die Ventil-Anordnung mit einem Beatmungsgerät. Die Ausgestaltung mit dem automatischen Lösen der Verbindung stellt sicher, dass bei gesperrter Absperreinheit keine Fluidverbindung zwischen dem Beatmungsgerät und dem Patienten hergestellt ist. Eine solche Fluidverbindung ist in der Regel unerwünscht. Der Vorgang, den Patienten vom Beatmungsgerät zu trennen, erfordert nur eine einzige manuell auszuführende Handlung, nämlich die Handlung, die Absperreinheit in die Verschlussposition zu bewegen. Diese Handlung bewirkt, dass die Ventil-Anordnung von der Fluidführungseinheit getrennt wird. Die geschlossene Absperreinheit stellt sicher, dass eine unerwünschte Fluidverbindung zwischen der Lunge des Patienten und der Umgebung gesperrt ist.

Die Erfindung betrifft weiterhin eine Beatmungs-Anordnung, welche ein Beatmungsgerät und mindestens eine Fluidführungseinheit umfasst, in einer Ausgestaltung mehrere Fluidführungseinheiten. Die oder jede Fluidführungseinheit steht wenigstens zeitweise mit dem Beatmungsgerät in einer Fluidverbindung. Jeder Fluidführungseinheit ist jeweils eine erfindungsgemäße Ventil-Anordnung zugeordnet. Jede Fluidführungseinheit lässt sich von der zugeordneten Ventil-Anordnung trennen. Die oder jede Ventil-Anordnung steht außerdem bevorzugt in einer Fluidverbindung mit einer patientenseitigen Koppeleinheit. Die Ventil-Anordnung stellt somit eine Trennstelle bereit. An dieser Trennstelle lässt sich die patientenseitige Koppeleinheit von dem Beatmungsgerät trennen. Aufgrund der Trennung werden die oben beschriebenen Vorteile erzielt.

Außerdem betrifft die Erfindung ein Computerprogramm, welches auf einen 3D-Drucker ausgeführt werden kann. Eine Rechnereinheit, welche das Computerprogramm auszuführen vermag, kann Bestandteil des 3D-Druckers sein oder räumlich vom 3D-Drucker entfernt sein. Wird das Computerprogramm auf der Rechnereinheit und damit auf dem 3D-Drucker ausgeführt, so steuert das Computerprogramm den 3D-Drucker an. Der vom Computerprogramm angesteuerte 3D-Drucker druckt dann eine erfindungsgemäße Ventil-Anordnung - oder wenigstens mindestens einen Bestandteil einer erfindungsgemäßen Ventil-Anordnung - aus.

Darüber hinaus betrifft die Erfindung einen 3D-Drucker, der dazu ausgestaltet ist, bei Ansteuerung durch ein Computerprogramm eine erfindungsgemäße Ventil-Anordnung - oder wenigstens mindestens einen Bestandteil einer erfindungsgemäßen Ventil-Anordnung - auszudrucken.

Im Folgenden wird die Erfindung anhand eines Ausführungsbeispiels beschrieben. Hierbei zeigen
- Figur 1: schematisch eine Anordnung umfassend ein Beatmungsgerät, ein Schlauchsystem und eine erfindungsgemäße Ventil-Anordnung;
- Figur 2: schematisch die Ventil-Anordnung in der Öffnungsstellung;
- Figur 3: schematisch die Ventil-Anordnung in der Verschlussstellung;
- Figur 4: einen rein mechanisch ausgestalteten Überdruckmechanismus mit einem Arretierkörper in der Arretierposition;
- Figur 5: den Überdruckmechanismus von Figur 4 mit dem Arretierkörper in der Freigabeposition;
- Figur 6: einen Überdruckmechanismus mit einem ansteuerbaren Stellglied für das Betätigungselement, wobei das Betätigungselement in der Verschlussposition ist;
- Figur 7: den Überdruckmechanismus von Figur 6 mit dem Betätigungselements in der Öffnungsposition;
- Figur 8: eine Ausgestaltung der Ventil-Anordnung in einer perspektivischen Darstellung mit der Absperreinheit in der Öffnungsstellung;
- Figur 9: die Ausgestaltung von Figur 8 von der anderen Seite;
- Figur 10: die Ausgestaltung aus der Betrachtungsrichtung von Figur 8 mit der Absperreinheit in der Verschlussstellung;
- Figur 11: die Ausgestaltung aus der Betrachtungsrichtung von Figur 9 mit der Absperreinheit in der Verschlussstellung;
- Figur 12: eine Querschnittsdarstellung durch die Ausgestaltung von Figur 8 bis Figur 11 mit der Absperreinheit in der Öffnungsstellung;
- Figur 13: eine Querschnittsdarstellung durch die Ausgestaltung von Figur 8 bis Figur 11 mit der Absperreinheit in der Verschlussstellung.

Im Ausführungsbeispiel wird die Erfindung in einer Fluidverbindung zur künstlichen Beatmung eines Patienten eingesetzt, optional in einem Beatmungskreislauf. Der Patient ist mit einer patientenseitigen Koppeleinheit, z.B. einem Beatmungstubus oder einem Katheter oder einer Atemmaske, verbunden. Die patientenseitige Koppeleinheit ist mittels eines Schlauchsystems mit einem Beatmungsgerät verbunden. Der Patient wird vom Beatmungsgerät mithilfe der Fluidverbindung künstlich beatmet. Das Beatmungsgerät führt Beatmungshübe aus.

Von Zeit zu Zeit ist es erforderlich, während der Beatmung des Patienten diese Fluidverbindung zwischen der patientenseitigen Koppeleinheit und dem Beatmungsgerät zu unterbrechen und die patientenseitige Koppeleinheit und damit den Patienten vom Beatmungsgerät zu trennen, beispielsweise weil eine Reinigung oder Wartung am Beatmungsgerät durchgeführt werden muss oder weil Betriebsmittel, insbesondere ein Filterelement, ergänzt oder ersetzt werden müssen oder weil der Patient von einem Beatmungsgerät zu einem anderen Beatmungsgerät transportiert wird. Der Patient wird also zeitweise von der künstlichen Beatmung abgekoppelt. In der Regel liegt die Zeitspanne, während welcher der Patient von der künstlichen Beatmung abgekoppelt ist, unterhalb von 3 Minuten. Der Patient bleibt auch während dieser Trennung mit der patientenseitigen Koppeleinheit verbunden.

Die Gefahr besteht, dass nach dieser Abkopplung die patientenseitige Koppeleinheit und damit die Lunge des Patienten unerwünscht in Fluidkommunikation mit der umgebenden Atmosphäre steht. Dies kann dazu führen, dass der verbleibende Luftdruck in der Lunge des Patienten (positive end-expiratory pressure, PEEP) abfällt. Bei manchen Patienten könnte dies dazu führen, dass die Lunge kollabiert oder eine Atelektase auftritt. Die erfindungsgemäße Ventil-Anordnung reduziert diese Gefahr und hält in vielen Fällen ausreichend lange einen restlichen Luftdruck (PEEP) in der Lunge aufrecht.

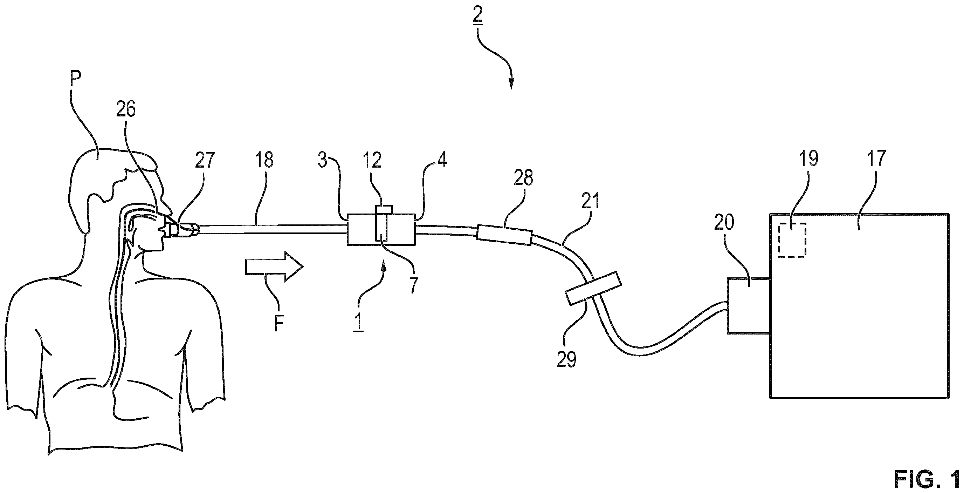

Figur 1 zeigt schematisch eine Beatmungs-Anordnung 2, welche wenigstens zeitweise einen Patienten P künstlich beatmet und folgende Bestandteile umfasst:
- ein Beatmungsgerät 17 mit einer optionalen Empfangseinheit 19,
- einen optionalen Atemluftfilter 20,
- einen geräteseitigen Atemluftschlauch 21,
- eine erfindungsgemäße Ventil-Anordnung 1 mit einer Absperreinheit 7, die sich öffnen und schließen lässt,
- einen patientenseitigen Atemluftschlauch 18,
- eine im Körper des Patienten P befindliches Teil 26 einer patientenseitigen Koppeleinheit, z. B. einen Endotrachealtubus oder eine Trachealkanüle oder einen Katheter,
- ein Mundstück 27 am patientenseitigen Ende des patientenseitigen Atemluftschlauchs 18,
- eine optionale Anschlusseinheit 28 im geräteseitigen Atemluftschlauch 21, die es ermöglicht, ein intrakorporales Gerät, z.B. einen Katheter oder ein Endoskop, in die Fluidverbindung einzuführen, und
- einen Filter 29 gegen Bakterien und Viren im geräteseitigen Atemluftschlauch 21.

Im Ausführungsbeispiel umfasst die patientenseitige Koppeleinheit den im Körper befindlichen Teil 26 und das Mundstück 27. Die patientenseitige Koppeleinheit kann auch eine Atemmaske umfassen.

Der patientenseitige Atemluftschlauch 18 stellt eine Fluidverbindung zwischen der patientenseitigen Koppeleinheit 26, 27 und der Ventil-Anordnung 1 her, der geräteseitige Atemluftschlauch 21 eine Fluidverbindung zwischen dem Beatmungsgerät 17 und der Ventil-Anordnung 1. Die beiden Atemluftschläuche 18 und 21 gehören zu dem Fluidführungssystem des Ausführungsbeispiels. Weil in einer Ausgestaltung auch ein Anästhesiemittel (Narkosemittel) durch die Fluidverbindung fließen kann, sind die Bestandteile der Ventil-Anordnung 1 bevorzugt aus einem Material, das nicht von Anästhesiemittel angegriffen wird.

Ein geräteseitiger Anschluss 4 der Ventil-Anordnung 1 ist lösbar mit dem geräteseitigen Atemluftschlauch 21 verbunden. Bevorzugt lässt sich der geräteseitige Atemluftschlauch 21 über den geräteseitigen Anschluss 4 schieben, der konusförmig sein kann. Der geräteseitige Anschluss 4 umfasst bevorzugt einen Schraub- oder Schnappverschluss, und der geräteseitige Atemluftschlauch 21 weist an einem patientenseitigen Ende ein entsprechendes Gegenstück auf. Bevorzugt umfasst der geräteseitige Atemluftschlauch 21
- zwei parallele Schläuche für das Einatmen (Inspiration) bzw. für das Ausatmen (Exspiration), die beide mit dem Beatmungsgerät 17 verbunden sind,
- einen einzigen Schlauch, der mit dem geräteseitigen Anschluss 4 verbunden ist, sowie
- ein T-Stück oder Y-Stück, das die parallelen Schläuche mit diesem einzelnen Schlauch verbindet.

Diese Ausgestaltung wird nicht in Figur 1 gezeigt.

Ein patientenseitiger Anschluss 3 der Ventil-Anordnung 1 ist lösbar oder fest mit dem patientenseitigen Atemluftschlauch 18 verbunden. Luft, die der Patient P ausatmet, fließt bei geöffneter Ventil-Anordnung 1 in eine Fließrichtung F durch den patientenseitigen Atemluftschlauch 18, die Ventil-Anordnung 1 und den geräteseitigen Atemluftschlauch 21 hindurch zum Beatmungsgerät 17. Bevorzugt lässt sich der patientenseitige Atemluftschlauch 18 in den patientenseitigen Anschluss 3 einschieben. Ein Beatmungshub des Beatmungsgeräts 17 bewirkt, dass Luft entgegen dieser Fließrichtung F vom Beatmungsgerät 17 zum Mundstück 27 und weiter in das Teil 26 fließt.

Möglich ist auch, dass die patientenseitige Koppeleinheit 26, 27 sich direkt mit dem patientenseitigen Anschluss 3 verbinden lässt und der patientenseitige Atemluftschlauch 18 nicht benötigt wird. Auch in diesem Falle ist eine Fluidverbindung zwischen der Ventil-Anordnung 1 und der patientenseitigen Koppeleinheit 26, 27 hergestellt.

Dank der Ventil-Anordnung 1 ist es möglich, die Fluidverbindung zwischen dem patientenseitigen Atemluftschlauch 18 und dem Beatmungsgerät 17 an einer Trennstelle zu trennen, ohne dass nach der Trennung eine Fluidkommunikation zwischen dem patientenseitigen Atem luftschlauch 18 und der Umgebung auftritt. Im Ausführungsbeispiel liegt diese Trennstelle zwischen den beiden Anschlüssen 3 und 4 der Ventil-Anordnung 1 oder an einem Anschluss 3, 4, sodass nach einer Trennung der patientenseitige Atemluftschlauch 18 von dem geräteseitigen Atemluftschlauch 21 getrennt wird. Die Ventil-Anordnung 1 reduziert die Gefahr, dass die Lunge des Patienten P kollabiert. Solange die Ventil-Anordnung 1 den patientenseitigen Atemluftschlauch 18 verschließt, ist es möglich, den Patienten P von dem Beatmungsgerät 17 zu trennen, ohne dass die unerwünschte Fluidkommunikation mit der Umgebung auftritt. Möglich ist es, nach der Trennung das Beatmungsgerät 17 zu reinigen oder zu warten und den Patienten P danach wieder mit dem Beatmungsgerät 17 zu verbinden. Möglich ist auch, den Patienten P zu einem anderen Gerät zu transportieren und anschließend den geräteseitigen Anschluss 4 mit einem Atemluftschlauch oder einer sonstigen Fluidführungseinheit dieses anderen Geräts zu verbinden.

Die Empfangseinheit 19 wird weiter unten erläutert.

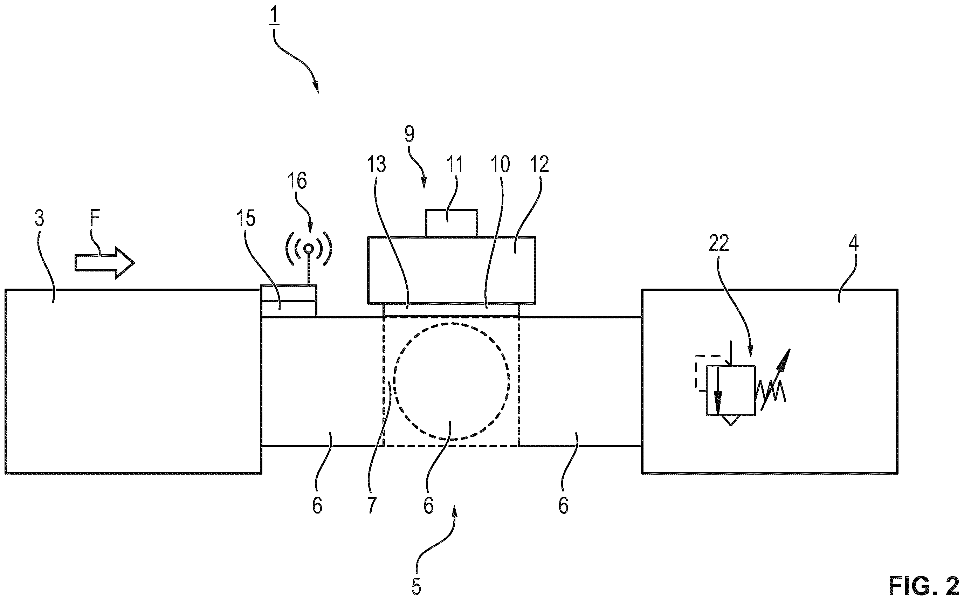

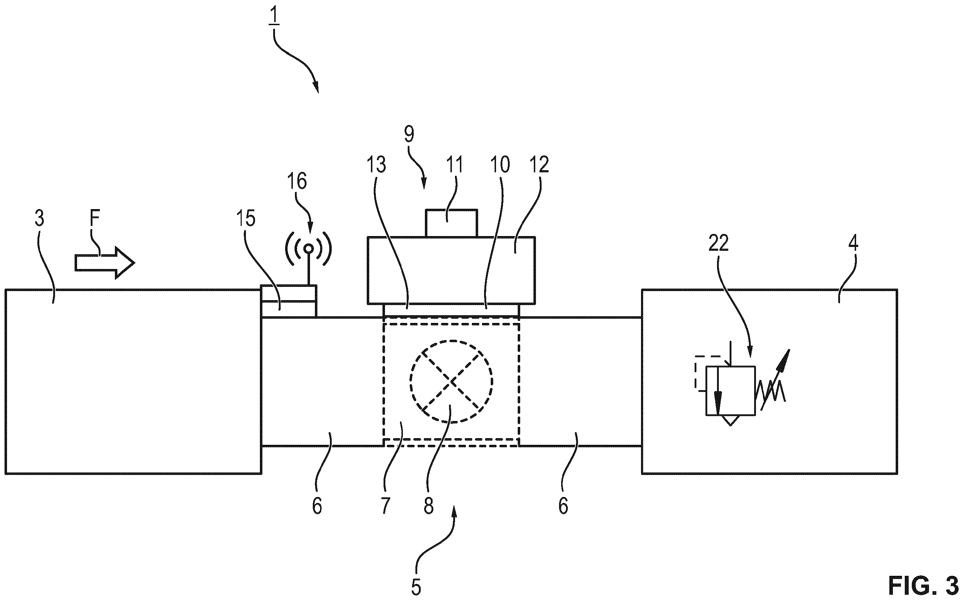

Figur 2 und Figur 3 zeigen schematisch die Ventil-Anordnung 1 des Ausführungsbeispiels. Eine röhrenförmige und bevorzugt starre Fluidführungseinheit 6 verbindet den patientenseitigen Anschluss 3 fluiddicht und bevorzugt drehfest mit dem geräteseitigen Anschluss 4. Ein manuell betätigbares Ventil 5 ist zwischen den beiden Anschlüssen 3 und 4 angeordnet und umfasst eine Absperreinheit 7, die sich zwischen einer Öffnungsstellung und einer Verschlussstellung hin und her bewegen lässt, und eine Betätigungseinheit 12 für die Absperreinheit 7.

In der Öffnungsstellung (Figur 2) ermöglicht die Absperreinheit 7, dass Fluid durch die Fluidführungseinheit 6 hindurchfließt, und beeinflusst dann bevorzugt den Fluss von Fluid durch die Ventil-Anordnung 1 nicht. Solange das Beatmungsgerät 17 den Patienten P künstlich beatmet, ist die Absperreinheit 7 in der Regel in der Öffnungsstellung. In der Verschlussstellung (Figur 3) verhindert die Absperreinheit 7, dass Fluid durch die Fluidführungseinheit 6 hindurch fließt, oder reduziert wenigstens den Volumenfluss durch die Fluidführungseinheit 6 verglichen mit der Öffnungsstellung.

Die Absperreinheit 7 umfasst bevorzugt eine starre flächige Klappe, ein elastisches Membran, eine Feder und / oder eine Kugel oder ein sonstiges geeignetes Absperrelement.

Mithilfe der Betätigungseinheit 12 des Ventils 5 kann ein Benutzer manuell die Absperreinheit 7 von der einen Stellung in die andere Stellung bewegen. Bevorzugt ist die Betätigungseinheit 12 mechanisch mit der Absperreinheit 7 verbunden und lässt sich zwischen einer Öffnungsposition und einer Verschlussposition hin- und herbewegen. Im Ausführungsbeispiel ist die Betätigungseinheit 12 fest mit der Absperreinheit 7 verbunden, und die beiden Elemente 7 und 12 können sich nicht relativ zueinander bewegen. Bevorzugt rastet die Betätigungseinheit 12 in jeder Endstellung (Öffnungsposition und Verschlussposition) ein, und zwar für einen Benutzer fühlbar ("haptisches Feedback") und bevorzugt auch hörbar ("akustisches Feedback"). Dank dieser Ausgestaltung merkt ein Benutzer, dass die Betätigungseinheit 12 tatsächlich in einer Endstellung ist.

Die Betätigungseinheit 12 kann auch als Fernbedienung ausgestaltet sein, die z.B. mittels eines Kabels oder per Funkwellen mit einem Stellglied für die Absperreinheit 7 verbunden ist. Ein Benutzer kann aus der Ferne die Fernbedienung 12 verwenden, um die Absperreinheit 7 zu öffnen und zu schließen.

Wird die Betätigungseinheit 12 aus der oder einer Öffnungsposition in eine Verschlussposition bewegt, so bewirkt diese Bewegung der Betätigungseinheit 12, dass die Absperreinheit 7 aus der oder einer korrespondierenden Öffnungsstellung in die Verschlussstellung überführt wird. Das Entsprechende gilt für eine Bewegung in die umgekehrte Richtung.

In einer Ausgestaltung ist ein nicht gezeigtes optionales Rückstellelement, beispielsweise eine Feder, bestrebt, die Betätigungseinheit 12 in die Öffnungsposition zu bewegen und in der Öffnungsposition zu halten. Die Betätigungseinheit 12 lässt sich gegen die Rückstellkraft dieses Rückstellelements aus der Öffnungsposition in die Verschlussposition bewegen. Diese Ausgestaltung reduziert die Gefahr, dass die Absperreinheit 7 ungewollt aus einer Öffnungsstellung in die Verschlussstellung überführt wird.

In einer Ausgestaltung sind die Absperreinheit 7 oder die Betätigungseinheit 12 mechanisch mit dem geräteseitigen Anschluss 4 gekoppelt. Sobald die Absperreinheit 7 in die Verschlussstellung überführt wird, wird automatisch der geräteseitige Atemluftschlauch 21 vom geräteseitigen Anschluss 3 gelöst und lässt sich abziehen. Beispielsweise wird eine Schnapphalterung des geräteseitigen Anschlusses 4 geöffnet. Bevorzugt bewirkt eine Bewegung der Betätigungseinheit 12 in die Verschlussposition, dass der geräteseitige Atemluftschlauch 21 automatisch abgetrennt wird. Diese Ausgestaltung reduziert die Gefahr, dass das Beatmungsgerät 17 bei hergestellter Fluidverbindung und geschlossenem Ventil 5 Beatmungshübe ausführt und diese Beatmungshübe zu einem starken Druckanstieg in dem verschlossenen geräteseitigen Atemluftschlauch 21 führen. Diese unerwünschte Situation könnte zu Beschädigungen am Beatmungsgerät 17 und / oder am Atemluftschlauch 21 führen.

Im Folgenden wird eine Ausgestaltung beschrieben, bei der die Absperreinheit 7 in genau eine Öffnungsstellung bewegt und in dieser arretiert werden kann. Daher wird der Begriff "die Öffnungsstellung" verwendet. Möglich sind auch verschiedene Öffnungsstellungen der Absperreinheit 7.

Eine in Figur 2 und Figur 3 nur schematisch dargestellte Arretiereinheit 10 hält in einem Öffnungs-Arretierzustand die Absperreinheit 7 in der Öffnungsstellung. In einem Verschluss-Arretierzustand hält die Arretiereinheit 10 die Absperreinheit 7 in der Verschlussstellung. Die Arretiereinheit 10 bewirkt also einen bistabilen Zustand der Absperreinheit 7: Die Absperreinheit 7 wird sowohl in der Öffnungsstellung als auch in der Verschlussstellung arretiert und kann dann nicht oder nur mit relativ großer Kraft - verglichen mit einem nicht arretierten Zustand - bewegt werden. Die Arretiereinheit 10 verhindert somit, dass die arretierte Absperreinheit 7 ungewollt bewegt wird. Dies gilt sowohl für die Absperreinheit 7 in der Öffnungsstellung als auch für die Absperreinheit 7 in der Verschlussstellung. Insbesondere verhindert die Arretiereinheit 10, dass die Absperreinheit 7 aufgrund von Vibrationen, Erschütterungen oder Berührungen oder mechanischen Kontakten ungewollt aus der einen Stellung in die andere Stellung oder in eine Zwischenstellung bewegt wird oder deshalb ungewollt geöffnet wird, weil der Patient P leicht hustet.

In einer Ausgestaltung ist die Arretiereinheit 10 direkt mit der Absperreinheit 7 verbunden. In einer anderen Ausgestaltung ist die Arretiereinheit 10 mit der Betätigungseinheit 12 mechanisch verbunden. Die Arretiereinheit 10 im Verschluss-Arretierzustand hält die Betätigungseinheit 12 in der Verschlussposition. Die Arretiereinheit 10 im Öffnungs-Arretierzustand hält die Betätigungseinheit 12 in der Öffnungsposition. Die auf diese Weise arretierte Betätigungseinheit 12 hält die Absperreinheit in der Verschlussstellung bzw. in der Öffnungsstellung.

In einer Ausgestaltung gehört zur Arretiereinheit 10 eine Rasteinheit, die beispielsweise eine Rastnase oder einen sonstigen Rastvorsprung und eine Rastaufnahme aufweist. Die Betätigungseinheit 12 rastet fühlbar und / oder hörbar ein, wenn sie eine Endposition erreicht, also die Verschlussposition oder die Öffnungsposition erreicht, und ist dann arretiert. Dies zeigt einem Benutzer an, dass eine Endposition erreicht ist. Eine mögliche visuelle Anzeige wird weiter unten beschrieben.

Bevorzugt lässt sich die Arretiereinheit 10 außerdem in einen Freigabezustand bringen. Wenn die Arretiereinheit 10 in dem Freigabezustand ist, so sind weder die Absperreinheit 7 noch die Betätigungseinheit 12 arretiert, die Betätigungseinheit 12 lässt sich betätigen, und die Absperreinheit 7 lässt sich aus der einen Stellung in die andere Stellung bringen, ohne eine arretierende Wirkung überwinden zu müssen. In einer Ausgestaltung umfasst die Arretiereinheit 10 ein Betätigungselement, z.B. das weiter unten beschriebene Sicherungselement 13.

Um die Arretiereinheit 10 aus einem Arretierzustand in den Freigabezustand zu überführen, muss ein Benutzer dieses Betätigungselement 13 zuvor betätigen.

Im Ausführungsbeispiel lassen sich die Absperreinheit 7 und die Betätigungseinheit 12 gemeinsam um 90° zwischen den beiden Endpositionen bewegen. Die Arretiereinheit 10 ist bevorzugt so ausgestaltet, dass sie einen Grenzwinkel ("Umkipp-Punkt") bewirkt: Solange der Winkel zwischen der aktuellen Stellung und der Öffnungsstellung der Absperreinheit 7 unterhalb dieses Grenzwinkels liegt, so ist die Arretiereinheit 10 bestrebt, die Absperreinheit 7 in die Öffnungsstellung zu bewegen und in dieser zu halten. Die Arretiereinheit 10 ist dann also im Öffnungs-Arretierzustand. Falls dieser Winkel oberhalb des Grenzwinkels liegt, so ist die Arretiereinheit 10 bestrebt, die Absperreinheit 7 in die Verschlussstellung zu bringen und in dieser zu halten, ist also im Verschluss-Arretierzustand.

Ein ebenfalls nur schematisch dargestelltes Sicherheitselement 13 hält in einer Ruheposition die Arretiereinheit 10 in dem Öffnungs-Arretierzustand. Eine Feder oder ein sonstiges geeignetes Rückstellelement übt eine Rückstellkraft aus, welche das Sicherheitselement 13 in dieser Ruheposition hält. Ein Benutzer kann das Sicherheitselement 13 gegen die Rückstellkraft betätigen. Nach einer Betätigung des Sicherheitselements 13 ist die Arretiereinheit 10 im Freigabezustand oder lässt sich in den Freigabezustand überführen. Diese Ausgestaltung reduziert weiter die Gefahr, dass die Absperreinheit 7 unbeabsichtigt oder versehentlich oder von allein aus der Öffnungsstellung in die Verschlussstellung bewegt wird. Um die Absperreinheit 7 in die Verschlussstellung zu bewegen, muss ein Benutzer zuerst das Sicherheitselement 13 gegen die Rückstellkraft aus der Ruheposition bewegen und dann die Betätigungseinheit 12 betätigen.

Möglich ist, dass der Patient P kräftiger hustet oder auf andere Weise kräftig ausatmet, während die Absperreinheit 7 in der Verschlussstellung arretiert ist. Diese Situation führt zu einem Druckanstieg am verschlossenen patientenseitigen Anschluss 3. Der Patient P könnte ernsthafte gesundheitliche Schäden an der Lunge erleiden, insbesondere ein Barotrauma, wenn dieser Druck nicht rasch genug abgebaut wird. Außerdem kann die Absperreinheit 7 durch einen Defekt oder ein Versehen in der Verschlussstellung verbleiben, obwohl der Patient P gefahrlos mit der Umgebung in einer Fluidkommunikation sein kann.

Um diese Gefahren für den Patienten P zu reduzieren, umfasst die Ventil-Anordnung 1 in einer Ausgestaltung einen optionalen Überdruckmechanismus 8, der in Figur 2 und Figur 3 ebenfalls nur schematisch dargestellt ist. Dieser Überdruckmechanismus 8 funktioniert wie folgt:

Wenn die Absperreinheit 7 in der Verschlussstellung ist, so ist die Arretiereinheit 10 in dem Verschluss-Arretierzustand und hält die Absperreinheit 7 in der Verschlussstellung. Wenn in dieser Situation am patientenseitigen Anschluss 3 ein Druck anliegt, der oberhalb einer vorgegebenen Druckschranke liegt, so wird der Überdruckmechanismus 8 aktiviert und überführt dann automatisch die Absperreinheit 7 in die Öffnungsstellung. Der Überdruckmechanismus 8 überwindet hierbei eine arretierende Wirkung, beispielsweise eine Rückstellkraft oder Arretierposition, welche die Arretiereinheit 10 in dem Verschluss-Arretierzustand ausübt, wobei diese arretierende Wirkung bestrebt ist, die Absperreinheit 7 in der Verschlussstellung zu halten.

Bevorzugt bewirkt die Bewegung, die der Druck am patientenseitigen Anschluss 3 und / oder der Überdruckmechanismus 8 hervorruft, dass die Arretiereinheit 10 in den Öffnungs-Arretierzustand überführt wird und nunmehr die Absperreinheit 7 in der Öffnungsstellung hält. Diese bevorzugte Ausgestaltung verhindert, dass die Absperreinheit 7 zwischen der Verschlussstellung und mindestens einer Zwischenstellung hin und her oszilliert, während der Patient P hustet. Vielmehr wird die Absperreinheit 7 in der Öffnungsstellung arretiert, sodass der Patient P ungestört husten und auch einatmen kann, ohne dass sich ein unerwünschter Druck aufbaut.

In einer Ausgestaltung wirkt der Überdruckmechanismus 8 direkt auf die Absperreinheit 7. In einer bevorzugten Ausgestaltung wirkt der Überdruckmechanismus 8 hingegen auf die Betätigungseinheit 12. Wenn die Absperreinheit 7 in der Verschlussstellung ist, so ist die Betätigungseinheit 12 in der Verschlussposition. Der aktivierte Überdruckmechanismus 8 bewegt die Betätigungseinheit 12 automatisch in die Öffnungsposition. Die mechanische Verbindung zwischen der Betätigungseinheit 12 und der Absperreinheit 7 bewirkt, dass die hervorgerufene Bewegung der Betätigungseinheit 12 auf die Absperreinheit 7 übertragen wird und die Absperreinheit 7 gegen die arretierende Wirkung der Arretiereinheit 10 in die Öffnungsstellung bewegt wird.

Wenn der Druck am patientenseitigen Anschluss 3 unterhalb dieser Druckschranke liegt, so nimmt der Überdruckmechanismus 8 keinen Einfluss auf die Absperreinheit 7 oder auf die Arretiereinheit 10. Insbesondere belässt der Überdruckmechanismus 8 die Arretiereinheit 10 in dem Verschluss-Arretierzustand, auch wenn der Druck, der am geräteseitigen Anschluss 4 anliegt, oberhalb der Druckschranke liegt.

Wie gerade dargelegt, arbeiten die Ventil-Anordnung 1 und insbesondere der optionale Überdruckmechanismus 8 abhängig von der vorgegebenen Druckschranke. Bevorzugt liegt die voreingestellte Druckschranke zwischen 50 mm Hg und 70 mm Hg, besonders bevorzugt bei 60 mm Hg. Viele Beatmungsgeräte und andere medizinische Geräte sind auf Ventile abgestimmt, die sich bei 60 mm Hg öffnen. Die Druckschranke liegt deutlich über dem endexspiratorischen Luftdruck, der in der Lunge aufrechterhalten werden soll (PEEP) und der bei Erwachsenen zwischen 4 mm Hg und 10 mm Hg liegt. Ein Benutzer kann mithilfe einer optionalen Justiereinheit 11 diese Druckschranke manuell verändern. Die Justiereinheit 11 umfasst beispielsweise ein Handrad sowie ein Anzeigemittel, beispielsweise eine Skala, welches die aktuell eingestellte Druckschranke anzeigt.

In einer Ausgestaltung ermöglicht die Justiereinheit 11 es, die Druckschranke kontinuierlich innerhalb eines Bereichs zu verändern. In einer bevorzugten Ausgestaltung lassen sich mithilfe der Justiereinheit nur endlich viele unterschiedliche Werte einstellen, bevorzugt mit einer Schrittweite von 10 mm Hg, besonders bevorzugt auf einen der vier Werte 40 mm Hg, 50 mm Hg, 60 mm Hg, 70 mm Hg. Bevorzugt rastet die Justiereinheit 11 in jeder Einstellung ein und hält die Druckschranke auf den eingestellten Wert. Dadurch wird verhindert, dass die Justiereinheit 11 versehentlich bewegt wird und dadurch die Druckschranke unbeabsichtigt verändert wird.

Optional umfasst die Ventil-Anordnung 1 zusätzlich zum Überdruckmechanismus 8 ein Überdruckventil 22, welches beispielsweise im geräteseitigen Anschluss 4 angeordnet ist. Falls in der Fluidverbindung zwischen dem Patienten P und dem Beatmungsgerät 17 ein Druck auftritt, der oberhalb einer vorgegebenen Überdruckschranke liegt, so öffnet sich dieses Überdruckventil 22 zeitweise und lässt Druck in die Umgebung aus, bevorzugt so lange, bis der Druck wieder unterhalb der Überdruckschranke liegt. Diese Überdruckschranke liegt bevorzugt oberhalb der Druckschranke für die Absperreinheit 7. Ein solcher Überdruck kann beispielsweise entstehen, wenn das Beatmungsgerät 17 aktiviert wird und die künstliche Beatmung aufnimmt, obwohl die Absperreinheit 7 in der Verschlussstellung ist, oder bei einem mechanischen Defekt, der zu einer Blockierung der Fluidverbindung führt.

Das optionale Überdruckventil 22 verhindert insbesondere, dass das Beatmungsgerät 17 bei der künstlichen Beatmung den Patienten P einem zu hohen Druck aussetzt. Weiterhin baut dieses Überdruckventil 22 einen Überdruck ab, wenn das Beatmungsgerät 17 bei geschlossenem Ventil 5 Beatmungshübe ausführt oder wenn der Patient P während der künstlichen Beatmung, also während die Absperreinheit 7 in der Öffnungsstellung ist, hustet oder auf andere Weise kräftig ausatmet. Bevorzugt lässt sich die Überdruckschranke dieses Überdruckventils 22 einstellen. Bevorzugt umfasst das Überdruckventil 22 ein Rückstellelement, welches bestrebt ist, das Überdruckventil 22 in eine Verschlussstellung zu bringen und in dieser Verschlussstellung zu halten. In einer Ausgestaltung öffnet das Überdruckventil 22 sich gegen die Kraft des Rückstellelements, wenn ein Druck oberhalb der Überdruckschranke anliegt, und schließt sich wieder, wenn der anliegende Druck unter eine kleinere weitere Überdruckschranke fällt. Bevorzugt öffnet das Überdruckventil 22 sich bei einem Druck oberhalb der Überdruckschranke, egal von welcher Seite der Druck anliegt.

Optional umfasst die Ventil-Anordnung 1 weiterhin einen Drucksensor 15 und eine Sendeeinheit 16. Der Drucksensor 15 misst mindestens bei geschlossener Absperreinheit 7 den Druck, der am patientenseitigen Anschluss 3 anliegt. Beispielsweise misst der Drucksensor 15 ein Maß für die Kraft, die vom patientenseitigen Anschluss 3 her auf die Absperreinheit 7 in der Verschlussstellung ausgeübt wird. Wenn die Absperreinheit 7 in der oder einer Öffnungsstellung ist, misst der Drucksensor 15 bevorzugt nicht einen Druck. Die Ventil-Anordnung 1 ermöglicht es daher in vielen Fällen, auf besonders einfache Weise einen Drucksensor zu realisieren.

Ein Messwert vom Drucksensor 15 wird an die Sendeeinheit 16 übermittelt. Die Sendeeinheit 16 übermittelt einen Messwert, den sie vom Drucksensor 15 empfangen hat, an mindestens einen räumlich entfernten Empfänger, und zwar bevorzugt drahtlos per Funkwellen. Der Druck-Messwert lässt sich auch dann übermitteln, wenn der geräteseitige Atemluftschlauch 21 von der Ventil-Anordnung 1 getrennt ist. Möglich ist auch, dass der Messwert kabelgebunden übertragen wird.

In Figur 1 wird beispielhaft eine Empfangseinheit 19 am Beatmungsgerät 17 gezeigt, die einen drahtlos übermittelten Druck-Messwert empfängt. Dieser Druck-Messwert wird beispielsweise auf einer nicht gezeigten Anzeigeeinheit angezeigt. Der oder ein räumlich entfernter Empfänger kann beispielsweise auch ein mobiles Gerät, z.B. ein Smartphone, ein Vibrationsarmband oder ein Empfänger einer stationären Anzeigeeinheit sein. Der Druck-Messwert lässt sich dafür verwenden, um dien Beatmungshübe des Beatmungsgeräts 17 zu regeln und hierbei mit der spontanen Atmung des Patienten P zu synchronisieren.

Anstelle des Drucksensors 15 oder zusätzlich zum Drucksensor 15 kann auch ein Ereignissensor vorgesehen sein, der automatisch das Ereignis detektiert, dass der Druck am patientenseitigen Anschluss 3 die vorgegebene Druckschranke überstiegen hat. Falls dieses Ereignis detektiert ist, wird ein Alarm ausgelöst. Als Reaktion auf diesen Alarm beobachtet beispielsweise ein Mensch den Patienten P oder prüft den Filter 29.

Wie bereits erwähnt, überführt der optionale Überdruckmechanismus 8 die Absperreinheit 7 dann automatisch aus der Verschlussstellung in die Öffnungsstellung, wenn der Druck am patientenseitigen Anschluss 3 oberhalb der Druckschranke ist. In einer Ausgestaltung ist diese Druckschranke so bemessen, dass der am patientenseitige Anschluss 3 anliegende Druck allein nicht ausreicht, um die arretierende Wirkung der Arretiereinheit 10 zu überwinden und die Absperreinheit 7 oder auch die Betätigungseinheit 12 gegen diese arretierende Wirkung zu bewegen. Daher umfasst der Überdruckmechanismus 8 bevorzugt ein Stellglied. Dieses Stellglied vermag die Absperreinheit 7 gegen die arretierende Wirkung in die Öffnungsstellung zu überführen. Das Stellglied wird aktiviert, wenn der anliegende Druck oberhalb der Druckschranke liegt.

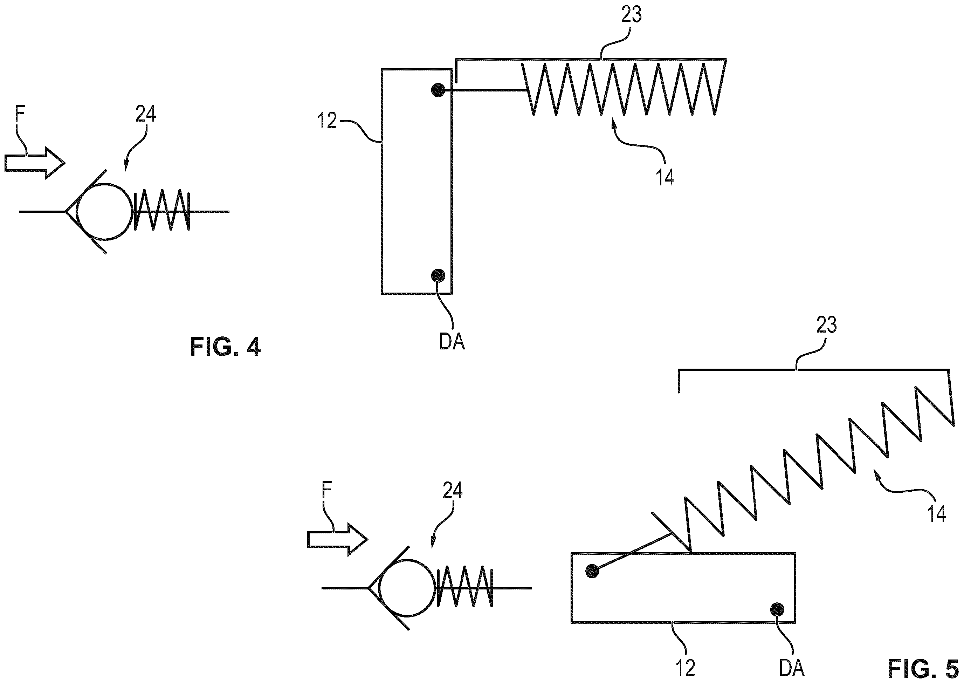

Figur 4 und Figur 5 zeigen schematisch eine beispielhafte rein mechanische Ausgestaltung des Überdruckmechanismus 8. Wie bereits erwähnt, bewirkt eine Bewegung der Betätigungseinheit 12 aus der Verschlussposition in die Öffnungsposition, dass die Absperreinheit 7 aus der Verschlussstellung in die Öffnungsstellung bewegt wird. Die Betätigungseinheit 12 ist mechanisch mit der Absperreinheit 7 verbunden, und zwar bevorzugt so, dass die Betätigungseinheit 12 sich nicht relativ zur Absperreinheit 7 bewegen kann. In Figur 4 und Figur 5 lässt sich die Betätigungseinheit 12 um die Drehachse DA bewegen.

Das Stellglied hat im gezeigten Beispiel die Form einer Druckfeder 14, welche in Figur 4 in einem gespannten Zustand und in Figur 5 in einem entspannten Zustand gezeigt wird. In Figur 4 ist die Betätigungseinheit 12 in der Verschlussposition, in Figur 5 in der Öffnungsposition. Die Druckfeder 14 wird zusammengedrückt und dadurch vorgespannt, wenn die Betätigungseinheit 12 von der Öffnungsposition in die Verschlussposition gedreht wird und dadurch die Absperreinheit 7 in die Verschlussstellung überführt. Eine Bewegung der Druckfeder 14 von dem gespannten Zustand in den entspannten Zustand bewegt die Betätigungseinheit 12 gegen die Arretierungswirkung der Arretiereinheit 10 aus der Verschlussposition in die Öffnungsposition. Diese Bewegung der Betätigungseinheit 12 bewirkt, dass die Absperreinheit 7 aus der Verschlussstellung in die Öffnungsstellung bewegt wird.

Ein schematisch gezeigter Arretierkörper 23 hält in einer Arretierposition die Druckfeder 14 in dem gespannten Zustand. Wenn die Betätigungseinheit 12 in die Verschlussposition gedreht und dadurch die Druckfeder 14 gespannt wird, so rastet der Arretierkörper 23 ein. Im gespannten Zustand wirkt die Druckfeder 14 nicht auf die Betätigungseinheit 12 ein. In einer Freigabeposition gibt der Arretierkörper 23 die Druckfeder 14 frei, was bewirkt, dass die Druckfeder 14 sich schlagartig entspannt und dadurch die Betätigungseinheit 12 um die Drehachse DA dreht und dadurch in die Öffnungsposition bewegt. Ein auslösendes Bauteil, z.B. ein Rückschlagventil 24, vermag den Arretierkörper 23 aus der Arretierposition in die Freigabeposition zu bewegen. Das auslösende Bauteil 24 ist so ausgestaltet, dass es den Arretierkörper 23 dann in die Freigabeposition bewegt, wenn der am patientenseitigen Anschluss 3 anliegende Druck die Druckschranke erreicht oder übersteigt. Bevorzugt sind das auslösende Element 24 und / oder die Druckfeder 14 im patientenseitigen Anschluss 3 angeordnet.

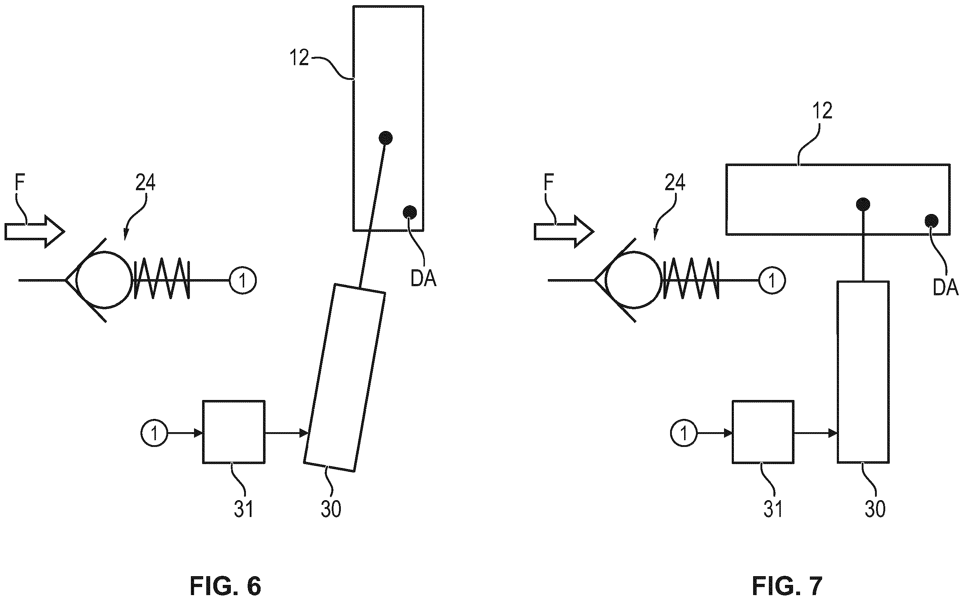

In einer anderen Ausgestaltung ist das Stellglied elektrisch oder pneumatisch oder hydraulisch ausgestaltet und lässt sich von außen oder vom auslösenden Element 24 ansteuern. Figur 6 und Figur 7 zeigen schematisch eine entsprechende Implementierung. Gleiche Bezugszeichen haben die gleichen Bedeutungen wie in Figur 4 und Figur 5.

Das auslösende Bauteil 24 erzeugt ein Signal, wenn der Druck am patientenseitigen Anschluss 3 oberhalb der Druckschranke liegt. Dieses Signal wird an ein Steuergerät 31 übermittelt. Das Steuergerät 31 steuert ein Stellglied 30 an, im vorliegenden Fall eine doppelt wirkende Kolben-Zylinder-Einheit. Das entsprechend angesteuerte Stellglied 30 dreht das Betätigungselement 12 um die Drehachse DA aus der Verschlussposition (Figur 6) in die Öffnungsposition (Figur 7) und wieder zurück aus der Öffnungsposition in die Verschlussposition.

In einer weiteren Implementierung empfängt das Steuergerät 31 einen Druck-Messwert, den der Drucksensor 15 bei geschlossener Absperreinheit 7 gemessen und übertragen hat. Das Steuergerät 31 steuert das Stellglied 30 an, wenn der gemessene Druck oberhalb der Druckschranke liegt. Das angesteuerte elektrische oder pneumatische oder hydraulische Stellglied 30 bewegt die Betätigungseinheit 12 aus der Verschlussposition in die Öffnungsposition oder direkt die Absperreinheit 7 aus der Verschlussstellung in die Öffnungsstellung. Die Ausgestaltungen mit dem mechanischen Überdruckmechanismus (Figur 4 und Figur 5) und mit dem ansteuerbaren Stellglied (Figur 6 und Figur 7) lassen sich kombinieren, beispielsweise um Redundanz zu schaffen.

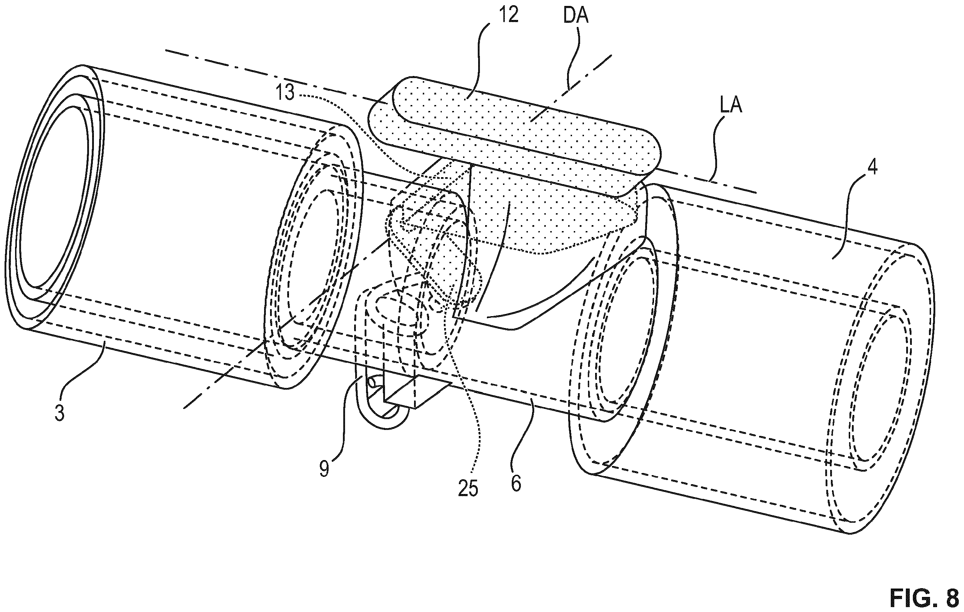

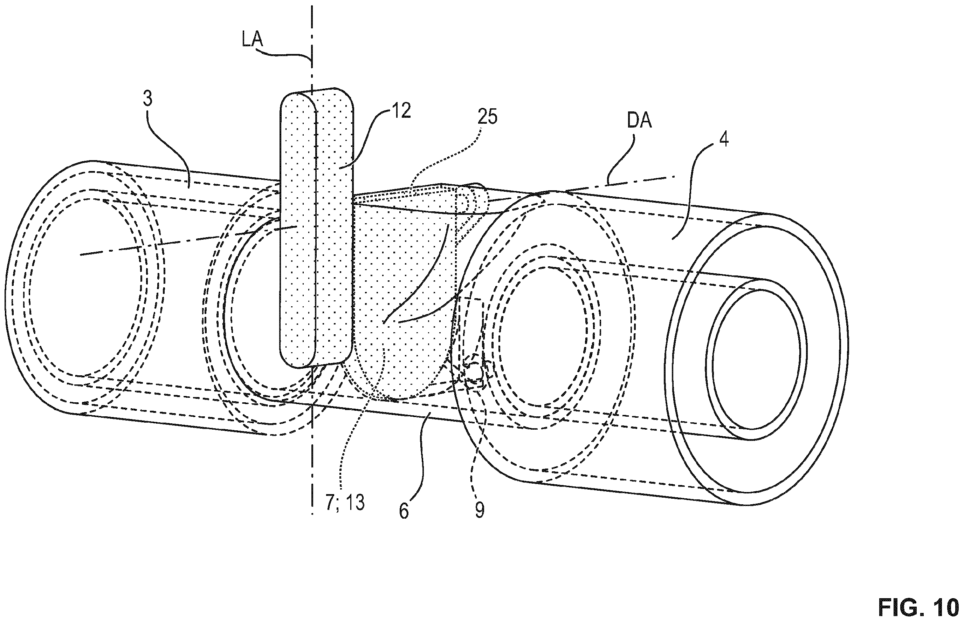

Figur 8 bis Figur 13 zeigen aus verschiedenen Betrachtungsrichtungen eine beispielhafte Ausgestaltung der Ventil-Anordnung 1, wobei der Überdruckmechanismus 8 nicht gezeigt wird. Figur 8, Figur 9 und Figur 12 zeigen die Betätigungseinheit 12 in der Öffnungsposition und somit die Absperreinheit 7 in der Öffnungsstellung, Figur 10, Figur 11 und Figur 13 die Betätigungseinheit 12 in der Verschlussposition und somit die Absperreinheit 7 in der Verschlussstellung.

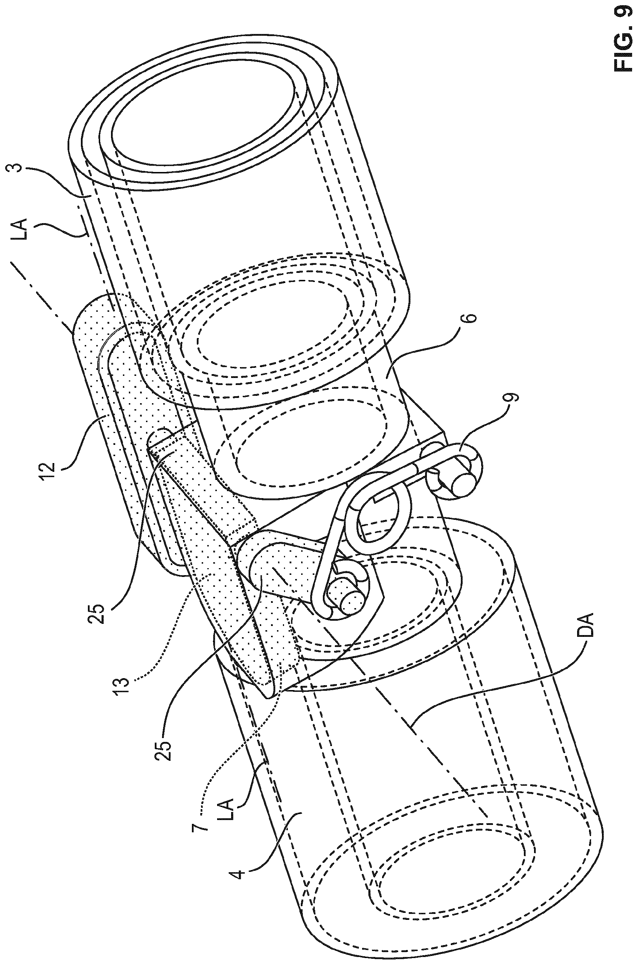

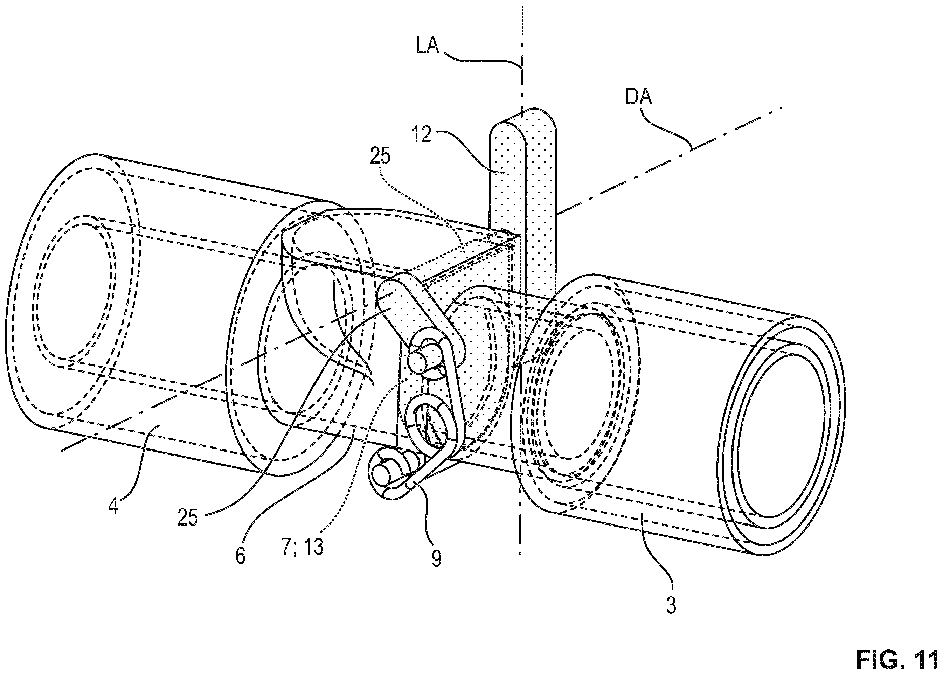

Die Betätigungseinheit 12 erstreckt sich entlang einer Längsachse LA und lässt sich in beiden Richtungen um die Drehachse DA drehen, und zwar um 90° zwischen zwei Endstellungen. Die Position der Betätigungseinheit 12 zeigt visuell an, ob sich die Absperreinheit 7 in der Öffnungsstellung oder in der Verschlussstellung befindet. Wenn die Absperreinheit 7 sich in der Öffnungsstellung befindet, so ist die Längsachse LA der Betätigungseinheit 12 parallel zur Längsachse der Fluidführungseinheit 6, wie dies in Figur 8 und Figur 9 gezeigt ist. Befindet sich die Absperreinheit 7 in der Verschlussstellung, so steht die Längsachse LA der Betätigungseinheit 12 senkrecht auf der Längsachse der Fluidführungseinheit 6, so wie dies in Figur 10 und Figur 11 gezeigt ist.

Dank dieser Ausgestaltung zeigt die Betätigungseinheit 12 auf intuitive Weise an, in welcher Stellung sich die Absperreinheit 7 aktuell befindet. Diese Ausgestaltung ist rein mechanisch und erfordert keine zusätzliche Anzeigeeinheit. Vielmehr fungiert die Betätigungseinheit 12 selbst als Anzeigeeinheit. Bevorzugt weist die Betätigungseinheit 12 außerdem eine andere Farbe als der Rest der Ventil-Anordnung 1 auf. Sie ist beispielsweise in einem kräftigen Rot gehalten. Möglich ist, dass die Oberfläche der Betätigungseinheit 12 mit einem phosphoreszierenden oder fluoreszierenden Material versehen ist, damit die Position der Betätigungseinheit 12 auch im Dunkeln oder bei schlechten Lichtverhältnissen gut erkannt werden kann.

In der gezeigten Ausgestaltung umfasst die Absperreinheit 7 ein flächiges Element, welches mechanisch mit der Betätigungseinheit 12 verbunden ist, beispielsweise eine Scheibe, die zwei parallele kreisförmige Stirnflächen oder eine ebene und eine konvexe Stirnfläche oder zwei parallele und nach außen gebogene Stirnflächen aufweist. In der Verschlussstellung versperrt dieses flächige Element die Fluidführungseinheit 6. In der Öffnungsstellung gibt dieses flächige Element die Fluidführungseinheit 6 frei. Bevorzugt ist die Mantelfläche des flächigen Elements an die Innenwand der Fluidführungseinheit 6 angepasst, sodass nur ein geringer Spalt zwischen dem flächigen Element und der Fluidführungseinheit 6 auftritt, wenn die Absperreinheit 7 in der Verschlussstellung ist. Diese Ausgestaltung erspart ein separates Dichtungselement. In vielen Fällen müsste ein solches Dichtungselement resistent gegen Anästhesiemittel sein.

In der Ausgestaltung mit der konvexen Stirnfläche kann das flächige Element der Absperreinheit 7 so ausgestaltet sein, dass die Absperreinheit 7 in der Öffnungsstellung bündig mit den Außenprofilen der beiden Anschlüsse 3 und 4 übereinstimmt. In der Ausgestaltung mit den beiden parallelen und nach außen gebogenen Stirnflächen kann das flächige Element so ausgestaltet sein, dass die Absperreinheit 7 in der Öffnungsstellung bündig mit der Innenwand der Fluidführungseinheit 6 übereinstimmt.

Die Arretiereinheit 10 umfasst ein mechanisches Verbindungselement 25 und eine Feder 9. Das Verbindungselement 25 verbindet mechanisch die Betätigungseinheit 12 mit der Feder 9. Die Feder 9 ist auf der einen Seite mit dem Verbindungselement 25 verbunden und stützt sich auf der anderen Seite an der röhrenförmigen Fluidführungseinheit 6 ab. Die Feder 9 hält die Betätigungseinheit 12 sowohl in der Öffnungsposition als auch in der Verschlussposition. Die Arretiereinheit 10 wirkt über das mechanische Verbindungselement 25 auf die Absperreinheit 7 und damit auf die Betätigungseinheit 12.

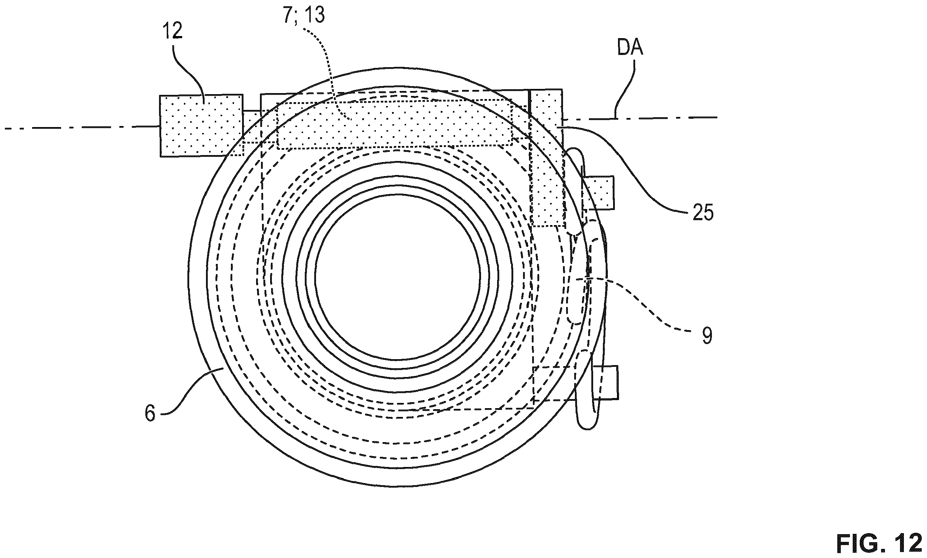

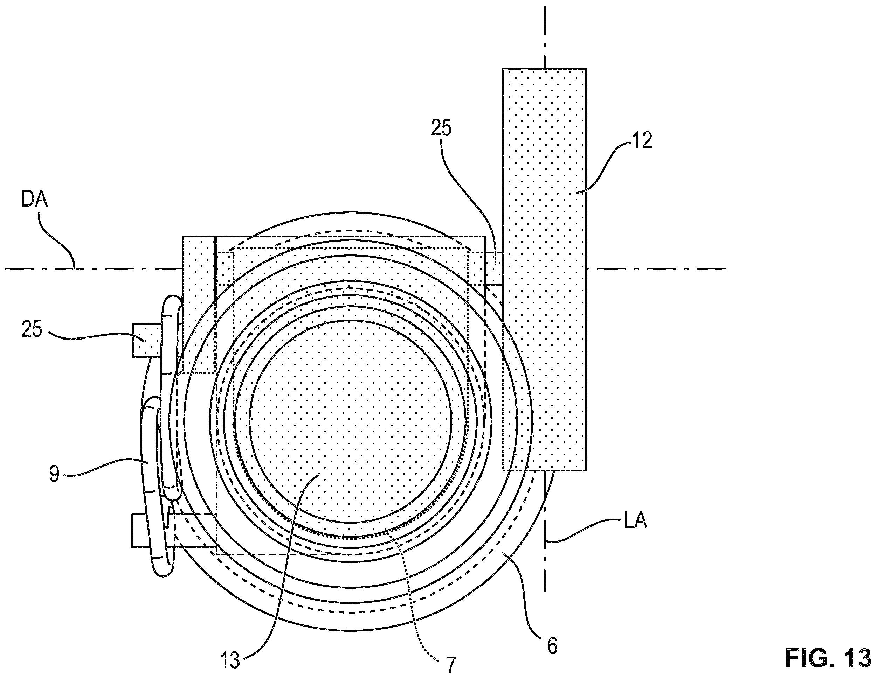

Figur 12 und Figur 13 zeigen die Ventil-Anordnung 1 in einer Querschnittsdarstellung. Die Fließrichtung F des Fluids steht senkrecht auf den Zeichenebenen von Figur 12 und Figur 13.

Bevorzugt ist das äußere Profil der Absperreinheit 7 an das innere Profil der bevorzugt röhrenförmigen Fluidführungseinheit 6 angepasst. In dieser Ausgestaltung verschließt die Absperreinheit 7 in der Verschlussstellung vollständig die Fluidführungseinheit 6. In einer Ausgestaltung ist an der Innenwand der Fluidführungseinheit 6 und / oder an der Außenwand der Absperreinheit 7 ein nicht gezeigtes Dichtungselement, beispielsweise ein Gummiring oder ein O-Ring, angebracht. Bevorzugt ist dieses Dichtungselement aus einem Material, welches nicht von einem Anästhesiemittel angegriffen wird. In einer anderen Ausgestaltung verschließt ausschließlich die Absperreinheit 7 die Fluidführungseinheit 6, bevorzugt formschlüssig, und ein separates Dichtungselement wird eingespart. Diese Ausgestaltung verhindert die Notwendigkeit, ein Dichtungselement an der Fluidführungseinheit 6 oder der Absperreinheit 7 zu befestigen.

Bevorzugt sind sowohl die Fluidführungseinheit 6 als auch die Absperreinheit 7 aus einem harten Kunststoff hergestellt, optional aus einem durchsichtigen Kunststoff. Dank der Ausgestaltung mit Kunststoff lassen die Teile 6 und 7 sich rasch fertigen, beispielsweise durch ein Spritzgießverfahren, und bei Bedarf rasch reinigen.

Bevorzugt ist die Absperreinheit 7 so angeordnet, dass die Absperreinheit 7 in der Öffnungsstellung sich vollständig außerhalb der Fluidführungseinheit 6 befindet. Bei dieser Ausgestaltung steht die gesamte Querschnittsfläche der Fluidführungseinheit 6 für den Fluss von Fluid zur Verfügung. Die Gefahr wird ausgeschlossen oder wenigstens reduziert, dass an der geöffneten Absperreinheit 7 eine Verwirbelung auftritt. Eine solche Verwirbelung kann zu einem stark variierenden Fluss von Fluid führen, und dies wiederum kann dazu führen, dass ein Volumenfluss-Sensor in der Fluidverbindung unzuverlässige Ergebnisse liefert und daher die spontane Atmung des Patienten P schlechter gemessen werden kann.

Außerdem behindert die geöffnete Absperreinheit 7 nicht den Vorgang, dass ein intrakorporales Gerät, z.B. ein Katheter oder Endoskop oder sonstiges Gerät, an der optionalen Anschlusseinheit 28 in den geräteseitigen Atemluftschlauch 21 eingeführt wird und durch die Ventil-Anordnung 1 hindurch in den patientenseitigen Atem luftschlauch 18 zum Patienten P hin geführt wird.

Wie in den Figuren zu sehen ist, wird die Absperreinheit 7 durch eine Drehbewegung um 90° zwischen der Verschlussstellung und der Öffnungsstellung hin und her bewegt. Dies sind die beiden Endpositionen, in die die Absperreinheit 7 des Ausführungsbeispiels gebracht werden kann. Die Arretiereinheit 10 vermag die Absperreinheit 7 in diesen beiden Endpositionen zu arretieren. In einer nicht gezeigten Alternative vermag die Arretiereinheit 10 die Betätigungseinheit 12 zusätzlich in einer Zwischenposition und damit die Absperreinheit 7 zusätzlich in einer Zwischenstellung zu arretieren. In dieser Zwischenposition tritt zwischen der Längsachse der Betätigungseinheit 12 und der Längsachse der Fluidführungseinheit 6 ein Winkel zwischen 0° und 90°, bevorzugt zwischen 30° und 60°, besonders bevorzugt gleich 45°, auf.

Auch in dieser Zwischenstellung ist ein Fluss von Fluid durch die Fluidführungseinheit 6 möglich. In einer Ausgestaltung bewirkt ein Druck am patientenseitigen Anschluss 3, der oberhalb der Druckschranke liegt, dass die Absperreinheit 7 aus der Verschlussstellung in die Zwischenstellung oder in die in Figur 12 gezeigte Öffnungsstellung bewegt wird, je nachdem wie groß der Druck ist. Weil die Absperreinheit 7 auch in der Zwischenstellung arretiert ist, wird verhindert, dass sie ungewollt wieder in die Verschlussstellung bewegt wird.

Die Absperreinheit 7 fungiert zugleich als das Sicherheitselement 13 und als ein Betätigungselement für die Arretiereinheit 10. Solange sich die Arretiereinheit 10 in einem Arretierzustand befindet, lässt sich die Betätigungseinheit 12 nur gegen die arretierende Wirkung, hier gegen eine große Federkraft, bewegen. Indem ein Benutzer das Sicherheitselement 13 betätigt, überführt er die Arretiereinheit 10 in einen nicht gezeigten Freigabezustand. In diesem Freigabezustand übt die Feder 9 eine geringere Kraft aus, und die Betätigungseinheit 12 lässt sich um 90° drehen. Diese Ausgestaltung reduziert insbesondere die Gefahr, dass die Absperreinheit 7 ungewollt in die Verschlussstellung überführt wird, und verringert den Kraftaufwand, der zum Bewegen der Betätigungseinheit 12 erforderlich ist.

**Bezugszeichenliste**

| | |
|---|---|
| 1 | Ventil-Anordnung, umfasst die Absperreinheit 7, die Betätigungseinheit 12, die Arretiereinheit 10, den Überdruckmechanismus 8 und die beiden Anschlüsse 3 und 4 |
| 2 | Beatmungs-Anordnung, umfasst das Beatmungsgerät 17, die Ventil-Anordnung 1, die Atemluftschläuche 21 und 18, die Anschlusseinheit 28 sowie die Atemluftfilter 20 und 29 |
| 3 | patientenseitiger Anschluss der Ventil-Anordnung 1, mit dem patientenseitigen Atemluftschlauch 18 verbunden |
| 4 | geräteseitiger Anschluss der Ventil-Anordnung 1, lösbar mit dem geräteseitigen Atemluftschlauch 21 verbunden |
| 5 | Ventil, umfasst die Absperreinheit 7 und die Betätigungseinheit 12 |
| 6 | röhrenförmige Fluidführungseinheit der Ventil-Anordnung 1, verbindet die Anschlüsse 3 und 4 miteinander |
| 7 | Absperreinheit, vermag in einer Verschlussstellung die Fluidführungseinheit 6 zu verschließen |
| 8 | Überdruckmechanismus, überführt die Absperreinheit 7 in die Öffnungsstellung, wenn der Druck am patientenseitigen Anschluss 3 oberhalb der Druckschranke liegt |
| 9 | Feder der Arretiereinheit 10 |
| 10 | Arretiereinheit, umfasst die Feder 9 und das Verbindungselement 25, hält in einem Öffnungs-Arretierzustand die Absperreinheit 7 in der Öffnungsstellung und in einem Verschluss-Arretierzustand die Absperreinheit 7 in der Verschlussstellung |
| 11 | Justiereinheit, ermöglicht es, die Druckschranke der Arretiereinheit 10 oder des Überdruckmechanismus 8 zu verändern |
| 12 | Betätigungseinheit zum Betätigen der Arretiereinheit 10, zwischen einer Öffnungsposition und einer Verschlussposition bewegbar, über das Verbindungselement 25 mit der Feder 9 verbunden |
| 13 | Sicherheitselement, hält in einer Ruheposition die Arretiereinheit 10 in dem Öffnungs-Arretierzustand |
| 14 | Stellglied in Form einer Druckfeder, vermag die Arretiereinheit 10 je nach deren Position in den Freigabezustand oder in den Öffnungs-Arretierzustand zu überführen |
| 15 | Drucksensor, misst bei geschlossener Absperreinheit 7 den Druck, der am patientenseitigen Anschluss 3 anliegt |
| 16 | Sendeeinheit, übermittelt einen Messwert des Drucksensors 15 an die Empfangseinheit 19 |
| 17 | Beatmungsgerät, über den Atemluftschlauch 21 mit der Ventil-Anordnung 1 verbunden |
| 18 | patientenseitiger Atemluftschlauch, mit dem patientenseitigen Anschluss 3 verbunden |
| 19 | Empfangsvorrichtung am Beatmungsgerät 17 |
| 20 | Atemluftfilter zwischen dem Atemluftschlauch 21 und dem Beatmungsgerät 17 |
| 21 | geräteseitiger Atem luftschlauch, lösbar mit dem geräteseitigen Anschluss 4 verbunden |
| 22 | Überdruckventil im geräteseitigen Anschluss 4 |
| 23 | Arretierkörper, welcher in der Arretierposition die Druckfeder 14 in einer gespannten Position hält, mit dem Rückschlagventil 24 verbunden |
| 24 | Rückschlagventil, welches den Arretierkörper 23 aus der Arretierposition in die Freigabeposition zu überführen vermag |
| 25 | mechanisches Verbindungselement zwischen der Feder 9 und der Betätigungseinheit 12 |
| 26 | im Körper des Patienten P angeordneter Teil der patientenseitige Koppeleinheit |
| 27 | Mundstück, mit dem Teil 26 verbunden, gehört zur patientenseitigen Koppeleinheit |
| 28 | Anschlusseinheit für einen Katheter oder ein Endoskop, im geräteseitigen Atemluftschlauch 21 angeordnet |
| 29 | Filter für Viren und Bakterien, im geräteseitigen Atemluftschlauch 21 angeordnet |
| 30 | Stellglied zum Drehen der Betätigungseinheit 12 um die Drehachse DA |
| 31 | Steuergerät, welches Signale von dem Rückschlagventil 24 empfängt und das Stellglied 30 ansteuert |
| DA | Drehachse der Betätigungseinheit 12 |
| F | Fließrichtung, in der Atemluft vom Patienten P durch die Ventil-Anordnung 1 hindurch zum Beatmungsgerät 17 fließt |
| LA | Längsachse der Betätigungseinheit 12 |
| P | Patient, mit dem patientenseitigen Atemluftschlauch 18 verbunden, wird vom Beatmungsgerät 17 künstlich beatmet |

## Patentansprüche

1. Ventil-Anordnung (1) für ein Fluidführungssystem (18, 21), welches eine Fluidverbindung zwischen einer patientenseitigen Koppeleinheit (26, 27) und einem Beatmungsgerät (17) herstellt oder herzustellen vermag,
wobei die Ventil-Anordnung (1)
- eine Absperreinheit (7),
- eine Arretiereinheit (10),
- einen patientenseitigen Anschluss (3) und
- einen geräteseitigen Anschluss (4)
umfasst,
wobei die Absperreinheit (7) zwischen mindestens einer Öffnungsstellung und einer Verschlussstellung hin und her bewegbar ist,
wobei
- eine Fluidkommunikation zwischen den beiden Anschlüssen (3, 4) der Ventil-Anordnung (1) hergestellt ist, wenn die Absperreinheit (7) in der oder einer Öffnungsstellung ist, und
- diese Fluidkommunikation unterbrochen oder wenigstens eingeschränkt ist, wenn die Absperreinheit (7) in der Verschlussstellung ist,
wobei die Arretiereinheit (10)
- in einem Öffnungs-Arretierzustand die Absperreinheit (7) in der oder einer Öffnungsstellung arretiert und
- in einem Verschluss-Arretierzustand die Absperreinheit (7) in der Verschlussstellung arretiert und
wobei die Ventil-Anordnung (1) dazu ausgestaltet ist,
dann, wenn die Absperreinheit (7) in der Verschlussstellung ist und außerdem am patientenseitigen Anschluss (3) ein Druck oberhalb einer vorgegebenen Druckschranke anliegt,
die Absperreinheit (7) automatisch und gegen eine arretierende Wirkung der Arretiereinheit (10) in die oder eine Öffnungsstellung zu bewegen.

2. Ventil-Anordnung (1) nach Anspruch 1,
**dadurch gekennzeichnet, dass**
die Ventil-Anordnung (1) so ausgestaltet ist,
dass eine automatische Bewegung der Absperreinheit (7) in die oder eine Öffnungsstellung aufgrund eines anliegenden Drucks oberhalb der Druckschranke bewirkt, dass
- die Arretiereinheit (10) in den Öffnungs-Arretierzustand überführt wird und
- die Absperreinheit (7) in dieser Öffnungsstellung arretiert.

3. Ventil-Anordnung (1) nach einem der vorhergehenden Ansprüche,
**dadurch gekennzeichnet, dass**
die Ventil-Anordnung (1) einen Überdruckmechanismus (8) umfasst,
welcher dazu ausgestaltet ist, automatisch
dann, wenn die Absperreinheit (7) in der Verschlussstellung ist und außerdem am patientenseitigen Anschluss (3) ein Druck oberhalb einer vorgegebenen Druckschranke anliegt,
die Absperreinheit (7) gegen eine arretierende Wirkung der Arretiereinheit (10) in die oder eine Öffnungsstellung zu bewegen.

4. Ventil-Anordnung (1) nach Anspruch 3,
**dadurch gekennzeichnet, dass**
die Ventil-Anordnung (1) zusätzlich eine Betätigungseinheit (12) umfasst, welche
- mechanisch mit der Absperreinheit (7) verbunden ist und
- dazu ausgestaltet ist, die Absperreinheit (7) zwischen der oder einer Öffnungsstellung und der Verschlussstellung hin und her zu bewegen,
wobei die Betätigungseinheit (12) bevorzugt manuell betätigbar ist,
wobei der Überdruckmechanismus (8) mechanisch mit der Betätigungseinheit (12) verbunden ist,
wobei der Überdruckmechanismus (8) dazu ausgestaltet ist, dann, wenn die Absperreinheit (7) in der Verschlussstellung ist und außerdem am patientenseitigen Anschluss (3) ein Druck oberhalb der Druckschranke anliegt,
die Betätigungseinheit (12) gegen die arretierende Wirkung der Arretiereinheit (10) zu bewegen,
wobei die bewirkte Bewegung der Betätigungseinheit (12) die Absperreinheit (7) in die oder eine Öffnungsstellung bewegt.

5. Ventil-Anordnung (1) nach Anspruch 3,
**dadurch gekennzeichnet, dass**
der Überdruckmechanismus (8) ein auslösendes Bauteil (24) und ein Stellglied (30) umfasst,
wobei das Stellglied (14, 30)
- aktivierbar ist und
- dazu ausgestaltet ist, bei einer Aktivierung die Absperreinheit (7) gegen eine arretierende Wirkung der Arretiereinheit (10) in die oder eine Öffnungsstellung zu bewegen, und
wobei das auslösende Bauteil (24) dazu ausgestaltet ist,
dann, wenn die Absperreinheit (7) in der Verschlussstellung ist und außerdem am patientenseitigen Anschluss (3) ein Druck oberhalb einer vorgegebenen Druckschranke anliegt, das Stellglied (14, 30) zu aktivieren.

6. Ventil-Anordnung (1) nach einem der vorhergehenden Ansprüche,
**dadurch gekennzeichnet, dass**
die Ventil-Anordnung (1) zusätzlich eine Betätigungseinheit (12) umfasst, welche mit der Absperreinheit (7) verbunden ist und
- zwischen einer Öffnungsposition, die mit der oder einer Öffnungsstellung der Absperreinheit (7) korrespondiert, und
- einer Verschlussposition, die mit der Verschlussstellung der Absperreinheit (7) korrespondiert,
hin- und her beweglich ist,
wobei die Betätigungseinheit (12) bevorzugt manuell betätigbar ist,
wobei eine Bewegung der Betätigungseinheit (12) aus der einen Position in die andere Position eine Bewegung der Absperreinheit (7) in die jeweils korrespondierende Stellung bewirkt und
wobei eine Bewegung der Absperreinheit (7) aus der Verschlussstellung in die Öffnungsstellung eine Bewegung der Betätigungseinheit (12) in die Öffnungsposition bewirkt.

7. Ventil-Anordnung (1) nach einem der vorhergehenden Ansprüche,
**dadurch gekennzeichnet, dass**
die Arretiereinheit (10)
- in einen Freigabezustand überführbar ist und
- in dem Freigabezustand eine Bewegung der Absperreinheit (7) freigibt und die Ventil-Anordnung (1) ein manuell betätigbares Sicherheitselement (13) umfasst,
welches in einer Ruheposition die Arretiereinheit (10) in dem Öffnungs-Arretierzustand hält,
wobei eine Betätigung des Sicherheitselements (13) bewirkt, dass die Arretiereinheit (10) in den Freigabezustand überführt wird.

8. Ventil-Anordnung (1) nach einem der vorhergehenden Ansprüche,
**dadurch gekennzeichnet, dass**
die Ventil-Anordnung (1) dazu ausgestaltet ist,
als Reaktion darauf, dass die Absperreinheit (7) aufgrund eines anliegenden Drucks oberhalb der Druckschranke automatisch in die oder eine Öffnungsstellung bewegt worden ist,
- einen Alarm zu erzeugen und
- den erzeugten Alarm auszugeben oder an einen räumlich entfernten Empfänger (19) zu übermitteln.

9. Ventil-Anordnung (1) nach einem der vorhergehenden Ansprüche,
**dadurch gekennzeichnet, dass**
die Ventil-Anordnung (1) einen Drucksensor (15) umfasst, welcher dazu ausgestaltet ist,
mindestens dann, wenn die Absperreinheit (7) in der Verschlussstellung ist, ein Maß für den am patientenseitigen Anschluss (3) anliegenden Druck zu messen.

10. Ventil-Anordnung (1) nach Anspruch 9,
**dadurch gekennzeichnet, dass**
die Ventil-Anordnung (1) eine Sendeeinheit (16) umfasst, welche dazu ausgestaltet ist,
- einen Messwert vom Drucksensor (15) zu empfangen und
- den empfangenen Messwert an einen räumlich entfernten Empfänger (19) zu übermitteln,
insbesondere per Funkwellen zu übermitteln.

11. Ventil-Anordnung (1) nach einem der vorhergehenden Ansprüche,
**dadurch gekennzeichnet, dass**
der geräteseitige Anschluss (4) lösbar mit einer Fluidführungseinheit (21) verbunden oder verbindbar ist,
wobei die Absperreinheit (7) und / oder die Betätigungseinheit (12) dergestalt mechanisch mit dem geräteseitigen Anschluss (4) gekoppelt sind,
dass eine Bewegung der Absperreinheit (7) in die Verschlussstellung bewirkt, dass die Fluidführungseinheit (21) automatisch von dem geräteseitigen Anschluss (4) getrennt wird.

12. Fluidführungs-Anordnung (1, 21) umfassend
- eine Ventil-Anordnung (1) gemäß Anspruch 11 und
- eine Fluidführungseinheit (21),
wobei der geräteseitige Anschluss (4) der Ventil-Anordnung (1)
- lösbar mit der Fluidführungseinheit (21) verbunden oder verbindbar ist und
- wenigstens zeitweise mit der Fluidführungseinheit (21) in einer Fluidverbindung steht.

13. Beatmungs-Anordnung (2) umfassend
- ein Beatmungsgerät (17),
- mindestens eine Fluidführungseinheit (21) und
- jeweils eine Ventil-Anordnung (1) nach einem der Ansprüche 1 bis 11 pro Fluidführungseinheit (21),
wobei die oder jede Fluidführungseinheit (21)
- lösbar mit der oder jeweils einer Ventil-Anordnung (1) verbunden ist und
- wenigstens zeitweise mit dem Beatmungsgerät (17) in einer Fluidverbindung steht.

14. Computerprogramm, welches auf einer Rechnereinheit einem 3D-Drucker ausführbar ist und dazu ausgestaltet ist, bei der Ausführung einen 3D-Drucker anzusteuern, wobei die Ansteuerung des 3D-Druckers bewirkt, dass der 3D-Drucker eine Ventil-Anordnung (1) nach einem der Ansprüche 1 bis 11 erzeugt.

15. 3D-Drucker, welcher dazu ausgestaltet ist, nach Ansteuerung durch ein Computerprogramm eine Ventil-Anordnung (1) nach einem der Ansprüche 1 bis 11 zu erzeugen.

## Claims

1. Valve arrangement (1) for a fluid-conducting system (18, 21) which produces or is capable of producing a fluid connection between a patient-side coupling unit (26, 27) and a respiratory device (17), wherein the valve arrangement (1) comprises
- a shut-off unit (7),
- a locking unit (10),
- a patient-side port (3), and
- a device-side port (4),
wherein the shut-off unit (7) is movable to and fro between at least one open position and a closure position,
wherein
- a fluid communication between the two ports (3, 4) of the valve arrangement (1) is produced when the shut-off unit (7) is in the or an open position, and
- said fluid communication is interrupted or at least restricted when the shut-off unit (7) is in the closure position,
wherein the locking unit (10)
- in an open locking state locks the shut-off unit (7) in the or an open position, and
- in a closure locking state locks the shut-off unit (7) in the closure position, and
wherein the valve arrangement (1) is configured so as,
whenever the shut-off unit (7) is in the closure position and, in addition, a pressure above a specified pressure limit prevails at the patient-side port (3),
to move the shut-off unit (7) into the or an open position automatically and counter to a locking effect of the locking unit (10).

2. Valve arrangement (1) according to Claim 1,
**characterized in that**
the valve arrangement (1) is configured in such a manner that an automatic movement of the shut-off unit (7) into the or an open position because of a prevailing pressure above the pressure limit causes
- the locking unit (10) to be transferred into the open locking state, and
- the shut-off unit (7) to lock in this open position.

3. Valve arrangement (1) according to either of the preceding claims,
**characterized in that**
the valve arrangement (1) comprises a pressure relief mechanism (8) which is configured so as automatically,
whenever the shut-off unit (7) is in the closure position and, in addition, a pressure above a specified pressure limit prevails at the patient-side port (3),
to move the shut-off unit (7) into the or an open position counter to a locking effect of the locking unit (10).

4. Valve arrangement (1) according to Claim 3,
**characterized in that**
the valve arrangement (1) additionally comprises an actuating unit (12) which
- is mechanically connected to the shut-off unit (7) and
- is configured to move the shut-off unit (7) to and fro between the or an open position and the closure position,
wherein the actuating unit (12) is preferably actuable manually,
wherein the pressure relief mechanism (8) is mechanically connected to the actuating unit (12),
wherein the pressure relief mechanism (8) is configured so as, whenever the shut-off unit (7) is in the closure position and, in addition, a pressure above the pressure limit prevails at the patient-side port (3),
to move the actuating unit (12) counter to the locking effect of the locking unit (10),
wherein the effected movement of the actuating unit (12) moves the shut-off unit (7) into the or an open position.

5. Valve arrangement (1) according to Claim 3,
**characterized in that**
the pressure relief mechanism (8) comprises a triggering component (24) and an adjustment member (30),
wherein the adjustment member (14, 30)
- is activatable and
- is configured so as, upon activation, to move the shut-off unit (7) into the or an open position counter to a locking effect of the locking unit (10), and
wherein the triggering component (24) is configured so as, whenever the shut-off unit (7) is in the closure position and, in addition, a pressure above a specified pressure limit prevails at the patient-side port (3), to activate the adjustment member (14, 30).

6. Valve arrangement (1) according to one of the preceding claims,
**characterized in that**
the valve arrangement (1) additionally comprises an actuating unit (12) which is connected to the shut-off unit (7) and is movable to and fro
- between an open position, which corresponds to the or an open position of the shut-off unit (7), and
- a closure position, which corresponds to the closure position of the shut-off unit (7),
wherein the actuating unit (12) is preferably manually actuable,
wherein a movement of the actuating unit (12) from the one position into the other position brings about a movement of the shut-off unit (7) into the respectively corresponding position, and
wherein a movement of the shut-off unit (7) from the closure position into the open position brings about a movement of the actuating unit (12) into the open position.

7. Valve arrangement (1) according to one of the preceding claims,
**characterized in that**
the locking unit (10)
- is transferrable into a release state, and
- in the release state releases a movement of the shut-off unit (7), and
the valve arrangement (1) comprises a manually actuatable safety element (13)
which, in an inoperative position, holds the locking unit (10) in the open locking state,
wherein an actuation of the safety element (13) causes the locking unit (10) to be transferred into the release state.

8. Valve arrangement (1) according to one of the preceding claims,
**characterized in that**
the valve arrangement (1) is configured so as,
in response to the shut-off unit (7) having been moved automatically into the or an open position because of a prevailing pressure above the pressure limit,
- to generate an alarm and
- to output the generated alarm or to transmit same to a spatially remote receiver (19).

9. Valve arrangement (1) according to one of the preceding claims,
**characterized in that**
the valve arrangement (1) comprises a pressure sensor (15) which is configured so as,
at least whenever the shut-off unit (7) is in the closure position, to measure an extent of the pressure prevailing at the patient-side port (3).

10. Valve arrangement (1) according to Claim 9,
**characterized in that**
the valve arrangement (1) comprises a transmission unit (16) which is configured so as
- to receive a measurement value from the pressure sensor (15) and
- to transmit the measurement value received to a spatially remote receiver (19),
in particular to transmit same by radio waves.

11. Valve arrangement (1) according to one of the preceding claims,
**characterized in that**
the device-side port (4) is releasably connected or connectable to a fluid-conducting unit (21),
wherein the shut-off unit (7) and/or the actuating unit (12) are/is mechanically coupled to the device-side port (4) in such a manner that a movement of the shut-off unit (7) into the closure position causes
the fluid-conducting unit (21) to be automatically separated from the device-side port (4).

12. Fluid-conducting arrangement (1, 21) comprising
- a valve arrangement (1) according to Claim 11, and
- a fluid-conducting unit (21),
wherein the device-side port (4) of the valve arrangement (1)
- is releasably connected or connectable to the fluid-conducting unit (21) and
- is at least temporarily fluidically connected to the fluid-conducting unit (21).

13. Respiratory arrangement (2) comprising
- a respiratory device (17),
- at least one fluid-conducting unit (21), and
- a respective valve arrangement (1) according to one of Claims 1 to 11 per fluid-conducting unit (21),
wherein the or each fluid-conducting unit (21)
- is releasably connected to the or in each case one valve arrangement (1) and
- is at least temporarily fluidically connected to the respiratory device (17).

14. Computer program which can be executed on a computer unit with a 3D printer and is configured so as, upon execution, to activate a 3D printer, wherein the activation of the 3D printer causes the 3D printer to produce a valve arrangement (1) according to one of Claims 1 to 11.

15. 3D printer which is configured so as, after activation by a computer program, to produce a valve arrangement (1) according to one of Claims 1 to 11.

## Revendications

1. Agencement de vanne (1) destiné à un système (18, 21) de guidage de fluides instaurant, ou apte à instaurer une liaison fluidique entre une unité de couplage (26, 27), située côté patient, et un appareil respiratoire (17),
lequel agencement de vanne (1) inclut
- une unité d'isolement (7),
- une unité d'arrêt (10),
- un raccord (3) situé côté patient et
- un raccord (4) situé côté appareil,
ladite unité d'isolement (7) pouvant accomplir des mouvements alternatifs entre au moins une position d'ouverture et une position d'obturation,
sachant
- qu'une communication par fluide est établie entre les deux raccords (3, 4) dudit agencement de vanne (1) lorsque l'unité d'isolement (7) occupe la, ou une position d'ouverture, et
- que cette communication par fluide est rompue, ou pour le moins restreinte, lorsque ladite unité d'isolement (7) occupe la position d'obturation,
sachant que l'unité d'arrêt (10)
- bloque l'unité d'isolement (7) dans la, ou dans une position d'ouverture dans un état de blocage à l'ouverture et
- bloque ladite unité d'isolement (7) dans la position d'obturation, dans un état de blocage à l'obturation, et
sachant que ledit agencement de vanne (1) est conçu
pour mouvoir l'unité d'isolement (7) vers la, ou vers une position d'ouverture, automatiquement et en opposition à un effet de blocage de l'unité d'arrêt (10), lorsque ladite unité d'isolement (7) occupe la position d'obturation et lorsque, par ailleurs, une pression excédant une limite de pression préétablie est appliquée au raccord (3) situé côté patient.

2. Agencement de vanne (1) selon la revendication 1,
**caractérisé par le fait que**
ledit agencement de vanne (1) est conçu
de telle sorte qu'un mouvement automatique de l'unité d'isolement (7) vers la, ou vers une position d'ouverture, suite à l'application d'une pression excédant la limite de pression, implique que
- l'unité d'arrêt (10) soit transposée à l'état de blocage à l'ouverture et
- bloque ladite unité d'isolement (7) dans cette position d'ouverture.

3. Agencement de vanne (1) selon l'une des revendications précédentes,
**caractérisé par le fait que**
ledit agencement de vanne (1) inclut un mécanisme de surpression (8) conçu
pour mouvoir automatiquement l'unité d'isolement (7) vers la, ou vers une position d'ouverture, en opposition à un effet de blocage de l'unité d'arrêt (10), lorsque ladite unité d'isolement (7) occupe la position d'obturation et lorsque, par ailleurs, une pression excédant une limite de pression préétablie est appliquée au raccord (3) situé côté patient.

4. Agencement de vanne (1) selon la revendication 3,
**caractérisé par le fait que**
ledit agencement de vanne (1) inclut, de surcroît, une unité d'actionnement (12) qui
- est reliée mécaniquement à l'unité d'isolement (7) et
- est conçue pour imprimer, à ladite unité d'isolement (7), des mouvements alternatifs entre la, ou une position d'ouverture et la position d'obturation,
ladite unité d'actionnement (12) pouvant, de préférence, être manœuvrée à la main,
le mécanisme de surpression (8) étant relié mécaniquement à ladite unité d'actionnement (12),
lequel mécanisme de surpression (8) est conçu
pour mouvoir ladite unité d'actionnement (12), en opposition à l'effet de blocage de l'unité d'arrêt (10),
lorsque l'unité d'isolement (7) occupe la position d'obturation et lorsque, par ailleurs, une pression excédant la limite de pression préétablie est appliquée au raccord (3) situé côté patient,
sachant que le mouvement de ladite unité d'actionnement (12), ainsi provoqué, meut ladite unité d'isolement (7) vers la, ou vers une position d'ouverture.

5. Agencement de vanne (1) selon la revendication 3,
**caractérisé par le fait que**
le mécanisme de surpression (8) comprend une pièce structurelle (24) à effet déclencheur, et un organe de manœuvre (30),
sachant que ledit organe de manœuvre (14, 30)
- peut être activé et
- est conçu pour mouvoir l'unité d'isolement (7) vers la, ou vers une position d'ouverture, lors d'une activation, en opposition à un effet de blocage de l'unité d'arrêt (10), et
sachant que ladite pièce structurelle (24), à effet déclencheur, est conçue pour activer ledit organe de manœuvre (14, 30) lorsque ladite unité d'isolement (7) occupe la position d'obturation et lorsque, par ailleurs, une pression excédant une limite de pression préétablie est appliquée au raccord (3) situé côté patient.

6. Agencement de vanne (1) selon l'une des revendications précédentes,
**caractérisé par le fait que**
ledit agencement de vanne (1) inclut, de surcroît, une unité d'actionnement (12) qui est reliée à l'unité d'isolement (7) et
peut accomplir des mouvements alternatifs
- entre un emplacement d'ouverture concordant avec la, ou avec une position d'ouverture de l'unité d'isolement (7) et
- un emplacement d'obturation concordant avec la position d'obturation de ladite unité d'isolement (7),
ladite unité d'actionnement (12) pouvant, de préférence, être manœuvrée à la main,
sachant qu'un mouvement de ladite unité d'actionnement (12), de l'un des emplacements à l'autre emplacement, provoque un mouvement de ladite unité d'isolement (7) vers la position respectivement concordante et
sachant qu'un mouvement de ladite unité d'isolement (7) vers ladite position d'ouverture, à partir de ladite position d'obturation, provoque un mouvement de ladite unité d'actionnement (12) vers l'emplacement d'ouverture.

7. Agencement de vanne (1) selon l'une des revendications précédentes,
**caractérisé par le fait que**
l'unité d'arrêt (10)
- peut être transposée à un état de libération et
- autorise un mouvement de l'unité d'isolement (7), dans ledit état de libération, et
ledit agencement de vanne (1) inclut un élément de sécurité (13) actionnable à la main
qui, en un emplacement de repos, maintient ladite unité d'arrêt (10) à l'état de blocage à l'ouverture,
un actionnement dudit élément de sécurité (13) ayant pour effet de transposer ladite unité d'arrêt (10) audit état de libération.

8. Agencement de vanne (1) selon l'une des revendications précédentes,
**caractérisé par le fait que**
ledit agencement de vanne (1) est conçu
- pour engendrer une alarme et
- émettre l'alarme engendrée, ou la transmettre à un récepteur (19) implanté à distance dans l'espace,
en réaction au fait que l'unité d'isolement (7) a été mue automatiquement vers la, ou vers une position d'ouverture suite à l'application d'une pression excédant la limite de pression.

9. Agencement de vanne (1) selon l'une des revendications précédentes,
**caractérisé par le fait que**
ledit agencement de vanne (1) inclut un capteur de pression (15) conçu pour mesurer un critère affecté à la pression, appliquée au raccord (3) situé côté patient,
au moins lorsque l'unité d'isolement (7) occupe la position d'obturation.

10. Agencement de vanne (1) selon la revendication 9,
**caractérisé par le fait que**
ledit agencement de vanne (1) inclut une unité émettrice (16) conçue
- pour recevoir une valeur de mesure émanant du capteur de pression (15) et
- transmettre la valeur de mesure reçue à un récepteur (19) implanté à distance dans l'espace,
notamment la transmettre par radio.

11. Agencement de vanne (1) selon l'une des revendications précédentes,
**caractérisé par le fait que**
le raccord (4) situé côté appareil est relié, ou peut être relié amoviblement à une unité (21) de guidage de fluides,
l'unité d'isolement (7), et/ou l'unité d'actionnement (12), étant couplée(s) mécaniquement audit raccord (4) situé côté appareil
de façon telle qu'un mouvement de ladite unité d'isolement (7), vers la position d'obturation, gouverne une dissociation automatique de ladite unité (21) de guidage de fluides d'avec ledit raccord (4) situé côté appareil.

12. Ensemble (1, 21) de guidage de fluides, incluant
- un agencement de vanne (1) conforme à la revendication 11 et
- une unité (21) de guidage de fluides,
sachant que le raccord (4) dudit agencement de vanne (1), situé côté appareil,
- est, ou peut être relié amoviblement à ladite unité (21) de guidage de fluides et
- est, au moins périodiquement, en liaison fluidique avec ladite unité (21) de guidage de fluides.

13. Dispositif respirateur (2) incluant
- un appareil respiratoire (17),
- au moins une unité (21) de guidage de fluides et
- un agencement de vanne (1) conforme à l'une des revendications 1 à 11, respectivement alloué à chaque unité (21) de guidage de fluides, sachant que l'unité, ou chaque unité (21) de guidage de fluides
- est raccordée amoviblement audit agencement de vanne (1), ou à un agencement respectif, et
- est, au moins périodiquement, en liaison fluidique avec ledit appareil respiratoire (17).

14. Programme informatique pouvant être exécuté sur une unité de calcul d'une imprimante 3D et conçu pour piloter une imprimante 3D, au cours de l'exécution, le pilotage de l'imprimante 3D ayant pour effet que ladite imprimante 3D produit un agencement de vanne (1) conforme à l'une des revendications 1 à 11.

15. Imprimante 3D conçue pour produire, après pilotage par un programme informatique, un agencement de vanne (1) conforme à l'une des revendications 1 à 11.
